# EUROPÄISCHE PATENTSCHRIFT

(11) **EP 1 051 198 B1**
(45) Veröffentlichungstag und Bekanntmachung des Hinweises auf die Patenterteilung: **26.05.2004**
(21) Anmeldenummer: 99902450.8
(22) Anmeldetag: 04.02.1999
(51) Int. Cl.: A61L 2/08, A23L 3/26, A23L 3/00, A23B 4/015, A23B 5/015, A23B 7/015, A23B 9/06

(54) **VERFAHREN UND ANLAGE ZUM BEHANDELN EINES GUTES MIT ELEKTRONENBESTRAHLUNG**
METHOD AND APPARATUS FOR TREATING AN ITEM
PROCEDE ET DISPOSITIF DE TRAITEMENT D'UN MATERIAU

(30) Priorität: 06.02.1998 AT 21698
(43) Veröffentlichungstag der Anmeldung: 15.11.2000
(73) Patentinhaber: Mediscan GmbH & Co. KG, 4550 Kremsmünster (AT)
(72) Erfinder: BIERBAUMER, Hans-Peter, A-4532 Rohr/Kremstal (AT)
(74) Vertreter: Secklehner, Günter, Dr.
(86) Internationale Anmeldenummer: PCT/AT1999/000031
(87) Internationale Veröffentlichungsnummer: WO 1999/039751

(56) Entgegenhaltungen:
- EP-A- 0 731 626
- WO-A-94/22162
- SADAT T. & VASSENAIX M.: "Use of a Linear Accelerator for Decontamination of Deboned Poultry Meat" RADIATION PHYSICS AND CHEMISTRY, Bd. 36, Nr. 5, 1990, Seiten 661-665, XP002111465
- Z.ZIMEK ET AL.: "EB INDUSTRIAL FACILITY FOR RADIATION STERILIZATION OF MEDICAL DEVICES" RADIATION PHYSICS AND CHEMISTRY, Bd. 42, Nr. 1-3, 1993, Seiten 571-572, XP002111464
- ALLEN J T ET AL: "A fully integrated 10 MeV electron beam sterilization system" RADIATION PHYSICS AND CHEMISTRY, Bd. 46, Nr. 4, 12. Oktober 1995 (1995-10-12), Seite 457-460 XP004051111 ISSN: 0969-806X
- Proceedings of the 10th International Meeting on radiation Processing, 11-16 May 1997, Anaheim, USA, veröffentlicht in "Radiation Physics and Chemistry", Vol 52,Nr 1-6 (1998-06-01), seite 491-494. HACKETT J L: "A state of the art electron beam sterilization facility - An integrated system" XP002112487
- Proceedings of the 10th International Meeting on radiation Processing, 11-16 May 1997, Anaheim, USA, veröffentlicht in "Radiation Physics and Chemistry", Bd 52,Nr 1-6 (1998-06-01), seite 469-473. Kamino Y: "10 MeV 25kW industrial electron LINAC" XP002112515
- MCKEOWN J ET AL: "Beam scanning for dose uniformity" RADIATION PHYSICS AND CHEMISTRY, Bd. 46, Nr. 46, 12. September 1995 (1995-09-12), Seite 1363-1372 XP004051299 ISSN: 0969-806X
- BURNS P ET AL: "The measurement, control, and validation of critical parameters in an electron beam sterilization facility" NUCLEAR INSTRUMENTS & METHODS IN PHYSICS RESEARCH, SECTION - B: BEAM INTERACTIONS WITH MATERIALS AND ATOMS, Bd. 113, Nr. 1, 1. Juni 1996 (1996-06-01), Seite 96-98 XP004007773 ISSN: 0168-583X
- MCLAUGHLIN W L ET AL: "Dosimetry systems for radiation processing" RADIATION PHYSICS AND CHEMISTRY, Bd. 46, Nr. 46, 12. September 1995 (1995-09-12), Seite 1163-1174 XP004051261 ISSN: 0969-806X

## Beschreibung

Die Erfindung betrifft ein Verfahren zum Verändern der Eigenschaften zumindest in einem Teilbereich und/oder zum Behandeln eines Gutes gemäß den Merkmalen des Anspruches 1, sowie eine Anlage zum Verändern der Eigenschaften zumindest in einem Teilbereich und/oder zum Behandeln eines Gutes durch Strahlung gemäß Anspruch 27.

Die strahlenchemische Behandlung von Produkten unterschiedlicher Spezies mit Strahlung aus den verschiedensten Quellen, z.B. γ-, Elektronenstrahlung, ist ein Verfahren, welches in den letzten Jahren immer mehr an Bedeutung gewinnt. Durch den Einsatz hochenergetischer Strahlung kann dieses Verfahren auch zur Sterilisation von Produkten eingesetzt werden. Bislang kam dabei aber vor allem zur Erreichung dieser Qualitätssteigerung neben der Begasung mit Giften wie Methylbromid oder Ethylenoxid, vor allem die Bestrahlung mit radioaktiven Isotopen, beispielsweise Kobalt 60 oder Cäsium 137, zum Einsatz. Zwar werden durch derartige Verfahren Mikroben, Bakterien und Keime in hohem Maße abgetötet, es ist jedoch dabei zu bedenken, daß die Strahlenquelle ständig aktiviert ist und nicht "einfach" abgeschaltet werden kann, sodaß bei Wartungsarbeiten in diesen Anlagen ständig eine potentielle Gefährdung des Wartungspersonals gegeben ist. Zudem ist diese Art von Strahlenkosmetik zumindest in der Welt der Medien nicht unumstritten und lassen sich derart behandelte Produkte nur schlecht verkaufen. Außerdem gehen wichtige Vitamine, Mineralien und Nährwerte verloren. Eine Bestrahlungsanlage dieser Art ist beispielsweise aus der US 3,564,241 A bekannt. Dieses Dokument behandelt eine Anlage, bei der Waren aller Art zur Sterilisation in Körbe geladen werden, welche auf über Kopf angebrachten Schienen geführt sind. Die Körbe werden auf diesen Schienen in das Innere einer Strahlungskammer verbracht, wo sie durch die gewählte Art der Verlegung der Führungsschiene sowohl von vorne als auch von hinten mit Kobalt 60 bestrahlt werden.

Um diese Probleme zu vermeiden, wurden die Bestrahlungsverfahren derart weiterentwickelt, daß anstelle von radioaktivem Material beheizte Kathoden als Strahlenquelle zum Einsatz kamen. Die aus der Kathode austretenden Elektronen werden zur Anode hin vorbeschleunigt und fokusiert und im Endeffekt auf die zu sterilisierenden Produkte gelenkt. Die Praxis zeigt, daß der Sterilisationsgrad der Produkte mit jenem vergleichbar ist, der durch radioaktive Strahlenbehandlung erzielt werden kann. Eine derartige Anlage ist aus der WO 94/22162 A bekannt. In ihrem Aufbau ist diese Anlage, abgesehen von der Art der Strahlenquelle, mit der aus oben angeführter US-A bekannten Anlage vergleichbar. Auch hier werden wiederum die Waren in Körben, welche auf einer Schiene geführt sind, durch den Behandlungsraum geführt. Nachteilig bei dieser Art der Beförderung der Produkte ist es, daß durch die gewählte Art des Transportsystems, im speziellen Teile der Transportkörbe, welche einer ständig wiederkehrenden Bestrahlung ausgesetzt sind, dieses einem erhöhten Verschleiß unterliegt.

Aus dem Dokument "Use of a Linear Accelerator for Decontamination of Deboned Poultry Meat" (Sadat T. & Vassenaix M., Radiation Physics and Chemistry, Bd. 36, Nr. 5, 1990, Seiten 661-665) ist eine Anlage zum Bestrahlen von Gütern mit Elektronenstrahlen bekannt, welche in einem Linearelektronenbeschleuniger erzeugt und beschleunigt werden. Dazu werden die zu behandelden Güter mit einem Fördersystem durch den Prozeßraum befördert. Das Fördersystem ist so ausgestaltet, daß die zu bestrahlenden Güter geradlinig in den Prozeßraum gebracht und die bestrahlten Güter über eine Art Labyrinth aus dem Prozeßraum an eine Ladestation übergeben werden. Darüber hinaus zeigt dieses Dokument die Verwendung verschiedener Dosimetermethoden auf

Aus dem Dokument "EB Industrial Facility for Radiation Sterilization of Medical Devices" (Z. Zimek et al., Radiation Physics and Chemistry, Bd. 42, Nr. 1-3, 1993, Seiten 571-572) ist eine Linearelektronenbeschleunigeranlage zur Sterilisation von medizinischen Einwegprodukten bekannt, wobei der Linearelektronenbeschleuniger oberhalb der Prozeßkammer angeordnet ist. Entsprechend den Angaben ist das Fördersystem mikroprozessorkontrolliert und kann damit die Geschwindigkeit des Prozeßförderers im Bereich zwischen 0,3 bis 7 m/min variiert werden.

Das Dokument "A state of the art electron beam sterilization facility - An integrated system" (Hackett J.L., Radiation Physics and Chemistry, Bd. 52, Nr. 1-6, 1998, Seiten 491-494) bezieht sich auf eine Elektronenbeschleunigeranlage mit getrennter Zu- und Abfuhr der zu bestrahlenden Güter in beziehungsweise aus dem Prozeßraum. Das Fördersystem ist aus mehreren Teilen zusammengesetzt und wird ein Großteil der Richtungsänderungen, insbesondere im Bereich der labyrinthartigen Zufuhr beziehungsweise Abfuhr der Güter mit Hilfe von bereits bekannten, kreisbogenförmigen Förderern durchgeführt. Im Bereich des Prozeßförderers erfolgt die Richtungsänderung nicht mit kreisbogenförmigen Förderern. Weiters ist bekannt, die Güter mir Barcodes zu versehen um eine Prozeßdatenüberwachung zu ermöglichen und sind dazu entsprechende Barcodelesegeräte angeordnet, mit deren Hilfe auch die ein- beziehungsweise zweiseitige Bestrahlung der Güter erkannt werden kann.

Schließlich beschreibt das Dokument "10 MeV 25 kW industrial electron LINAC" (Kamino Y., Radiation Physics and Chemistry, Bd. 52, Nr. 1-6, 1998, Seiten 469-473) ebenfalls einen LINAC für die Sterilisation von medizinischen Produkten. Es wir darin eine zumindest zum Zeitpunkt des Erscheinens dieses Dokumentes kommerziell nicht erhältliche Elektronenbeschleunigeranlage behandelt. Die Bestrahlung erfolgt in bezug auf die Produkte wiederum von oben. Es wird kein Beschleunigersystem mit stehender Welle verwendet.

Der vorliegenden Erfindung liegt die Aufgabe zugrunde, ein Verfahren sowie eine Anlage zum Verändern der Eigenschaften zumindest in einem Teilbereich und/oder zum Behandeln eines Gutes durch Bestrahlung zu schaffen, um eine gleichmäßige Beaufschlagung mit Elektronen über einen möglichst großen Volumenbereich zu ermöglichen.

Diese Aufgabe der Erfindung wird durch die Merkmale des Anspruches 1 gelöst. Vorteilhaft ist dabei, daß es mit dem derartigen Verfahren erstmals möglich ist, Güter unterschiedlichster Art, beispielsweise Lebensmittel wie Gewürze, Wasser oder dgl., Werkstoffe wie z.B. Kunststoffe, Keramiken, Metalle oder dgl., so zu bestrahlen, daß damit eine bedeutende Qualitätsverbesserung erreicht werden kann. Von Vorteil ist dabei auch, daß die Elektronenstrahlung mit einer vordefinierbaren Frequenz gepulst werden kann, wodurch die Homogenität der Dosisverteilung im zu bestrahlenden Gut günstig beeinflußt werden kann.

Durch die vorteilhaften Weiterbildungen nach Anspruch 2 ist es einerseits möglich, eine einfache und im Betrieb sichere Technologie zur Beschleunigung der Elektronen einzusetzen und andererseits den Effekt der Beschleunigung zu verstärken.

Durch die Ausbildung nach Anspruch 3 wird auf vorteilhafte Weise eine weitere Steigerung der Homogenität der Dosisverteilung in dem zu bestrahlenden Gut erreicht.

Mit den Ausgestaltungen nach Anspruch 4 ist es auf vorteilhafte Weise möglich, die jeweils erforderliche Strahlendosis auf das zu bestrahlende Gut und im speziellen auf dessen Abmessungen abzustimmen.

Nach einer Weiterbildung nach Anspruch 5 wird eine vorteilhafte Verkürzung des Bestrahlungsprozesses und somit ein höherer Gutumsatz erreicht.

Durch die Ausbildungen nach Anspruch 6 ist es auf vorteilhafte Weise möglich, bei einer hohen Produktionsrate die zu verabreichende Dosis auf die jeweiligen Eigenschaften der zu bestrahlenden Güter abzustimmen.

Durch eine Verfahrensvariante nach Anspruch 7 ist es möglich, die während der Bestrahlung verabreichte Dosis mittels radiochromen Filmmaterials für eine spätere Auswertung festzuhalten und diese Daten einer späteren Kontrolle zugänglich zu machen.

Durch vorteilhafte Weiterbildungen nach den Ansprüchen 8 und 9 wird ein hoher Automatisierungsgrad des Bestrahlungsprozesses erreicht.

Vorteilhaft ist aber auch eine Weiterbildung nach Anspruch 10, da damit eine zusätzliche Kontroll- und Überwachungsroutine geschaffen wird.

Durch die Ausbildung nach Anspruch 11 ist mit Vorteil eine universelle Anwendbarkeit des Verfahrens für Güter unterschiedlichster Art möglich.

Gemäß einer Ausbildung nach Anspruch 12 können die zu bestrahlenden Güter auf einfache Art individualisiert werden und ist so ein Nachvollzug des Bestrahlungsprozesses zu einem späteren Zeitpunkt auf einfache Weise möglich.

Dabei erweist sich eine Ausgestaltung nach Anspruch 13 als vorteilhaft, da damit mit einem hohen Sicherheitsgrad ein zur Kontrolle der verabreichten Strahlendosis verwendetes Dosimeter eindeutig dem Gut, an dem dieses Dosimeter angebracht war, zugeordnet werden kann.

Nach den vorteilhaften Weiterbildungen gemäß Anspruch 14 und 15 wird eine Produktivitätssteigerung erreicht.

Durch die Ausgestaltung nach Anspruch 16 ist es auf vorteilhafte Weise möglich, Güter entsprechend ihrem Behandlungsgrad dem Beschleunigerraum zuzuführen.

Dabei erweist sich eine Ausgestaltung nach Anspruch 17 als vorteilhaft, da damit eine weitere Steigerung des Automatisierungsgrades des Produktionsprozesses erfolgen kann.

Vorteilhaft sind weiters Ausgestaltungen nach Anspruch 18. wonach eine Mehrfachbestrahlung eines Gutes durchgeführt werden kann, sodaß die Betriebsparameter der Beschleunigereinheit auf einem Niveau gehalten werden können, die einen störungsfreien Lauf über einen langen Zeitraum gewährleisten.

Nach einer Ausführungsvariante gemäß Anspruch 19 können fertigbestrahlte Güter einer Verladezone zugeführt werden.

Die Ausgestaltung nach Anspruch 20 stellt eine wirkungsvolle Maßnahme zur Verfügung, mit der die im Gut deponierte Dosis definiert werden kann.

Bei der Ausgestaltung nach Anspruch 21 ist von Vorteil, daß die zu verabreichende minimale Strahlendosis über signifikante Punkte im bzw. am Gut ermittelt wird, sodaß die im eigentlichen Produktionsprozeß verwendeten Parameter für die Bestrahlung die größtmögliche Gutschonung gewährleisten.

Vorteilhaft ist auch eine Ausbildung nach Anspruch 22, wonach die Verfahrensparameter in Abhängigkeit von der zu verabreichenden Strahlendosis eingestellt, geregelt und kontrolliert werden können.

Vorteilhaft ist auch eine Ausführungsform nach Anspruch 23, wonach ein Mittel zur Verfügung gestellt wird, welches eine wiederkehrende Kontrolle der Stabilität des Bestrahlungsprozesses ermöglicht.

Durch die Weiterbildung nach Anspruch 24 kann die Kontrolle der Stabilität des Bestrahlungsprozesses individuell und zufallsverteilt erfolgen.

Es ist aber auch eine Weiterbildung des Verfahrens, wie im Anspruch 25 beschrieben, möglich, bei der die Kontrolle des Bestrahlungsprozesses durch z.B. Visualisierung auf einem Bildschirm praktisch auf einen Blick erfolgen kann.

Schließlich ist bei einer Weiterbildung nach Anspruch 26 von Vorteil, daß damit eine automatische Optimierung und Anpassung der Verfahrensparameter an das jeweilige zu behandelnde Gut möglich ist.

Die Aufgabe der Erfindung wird aber auch durch die Merkmale im Anspruch 27 gelöst. Dabei erweist sich als vorteilhaft, daß im Vergleich zu herkömmlichen Anlagen die Beschränkung auf eine maximale Gutgröße weitestgehend aufgehoben ist und zudem der Elektronenstrom aufgrund von Zwischenräumen zwischen einzelnen Gütern nicht ungenützt bleibt bzw. daß der Elektronenstrom nicht unterbrochen werden muß.

Durch die Weiterbildung nach Anspruch 28 kann eine derartige erfindungsgemäße Anlage auf einfache Weise in eine Gesamtanlage für einen Produktionsprozeß integriert werden. Zudem können mit dieser Ausführung auf einfache Weise Vorkehrungen geschaffen werden, die einen schnelleren und sicheren Abtransport der fertigen Güter, beispielsweise durch einen LKW, erlauben.

Vorteilhaft ist auch eine weitere Ausführungsform nach Anspruch 29, wonach die zu bestrahlenden Güter in einem bestimmten, vordefinierbaren Winkel an der Beschleunigereinheit vorbeitransportiert werden, sodaß in der Folge eine ungleiche Dosisverteilung in den Gütern nicht zu erwarten ist.

Vorteilhaft ist weiters eine Ausbildung nach Anspruch 30, da damit eine Kontaminierung der fertig bestrahlten Güter weitestgehend ausgeschlossen werden kann.

Nach einer anderen Ausführungsvariante gemäß Anspruch 31 können Elektronen auf einfache Art auf eine hohe Geschwindigkeit beschleunigt werden und steht somit immer ein ausreichendes Depot an Elektronen hoher Energie zur Verfügung.

Durch die Weiterbildungen nach Anspruch 32 wird erreicht, daß der Elektronenstrahl einen großen Teil der Oberfläche der zu bestrahlenden Güter überstreichen kann.

Gemäß einer Ausbildung, wie in Anspruch 33 beschrieben, können die zu bestrahlenden Güter in nur einem Umlauf endgefertigt werden. Gleichzeitig ist es möglich, eine derartige Elektronenaustrittsvorrichtung zur Beschleunigung der Elektronen heranzuziehen.

Von Vorteil ist auch eine Ausbildung nach Anspruch 34, da damit Güter mit großen Abmessungen bestrahlt werden können, ohne daß Qualitätseinbußen der Bestrahlung zu erwarten sind.

Von Vorteil sind aber auch Ausbildungen nach Anspruch 35, da damit eine homogene Verteilung der deponierten Strahlendosis erreicht werden kann.

Durch die Weiterbildungen nach den Ansprüchen 36 bis 37 ist mit Vorteil ein hoher Produktumsatz und somit eine verkürzte Produktionsdauer möglich.

Nach den Ausführungsformen gemäß Anspruch 38 wird erreicht, daß bei konstanten Betriebsparametern für die Beschleunigereinheit die Homogenität der verabreichten Dosis im Gut großteils gewährleistet werden kann.

Gemäß einer Ausbildung nach Anspruch 39 ist es möglich, das Fördersystem aus kostengünstigen und wartungsfreundlichen Einzelbauteilen zusammenzustellen.

Die Aufteilung des Gesamtfördersystems in einzelne Teile, wie in Anspruch 40 beschrieben, ermöglicht mit Vorteil, diese Teile auf die jeweilige Strahlenbelastung durch den Elektronenbeschleuniger abzustimmen.

Durch die Weiterbildungen nach den Ansprüchen 41 bis 43 ist mit Vorteil eine Individualisierung der Güter zu erreichen, sodaß damit ein wirkungsvolles Mittel für das Qualitätsmanagement zur Verfügung steht.

Dabei erweisen sich Ausgestaltungen nach den Ansprüchen 44 und 45 als vorteilhaft, wonach die Individualität der Güter automatisch erfaßt werden kann.

Gemäß den Ausbildungen nach den Ansprüchen 46 und 47 wird ein Unterbrechen des Gutstromes durch Fluktuationen im Aufgabebereich weitestgehend aufgehoben.

Dabei erweist sich eine Ausführung nach Anspruch 48 als vorteilhaft, da mit einer derart ausgebildeten Betätigungseinrichtung für einen Gutstop eine nicht wartungsintensive und im Dauerbetrieb stabile Variante zur Verfügung steht.

Gemäß den Ausbildungen nach den Ansprüchen 49 und 50 ist es mit Vorteil und auf einfache Weise möglich, den Behandlungsgrad der Güter festzustellen und somit den Wirkungsgrad der Gesamtanlage zu verbessern.

Durch die Ausbildung des Prozeßförderers als Drahtgeflechtgurt gemäß Anspruch 51 kann eine hohe Betriebssicherheit des Prozeßförderers erreicht werden.

Es ist aber auch eine Ausbildung, wie im Anspruch 52 beschrieben, möglich, wonach mit Vorteil die Umwelt wegen der Schonung der Primärressourcen zur Erzeugung von Wärme geschont wird.

Schließlich bietet eine Ausbildung nach Anspruch 53 den Vorteil, daß im Inneren der Anlage ein für den Menschen verträgliches Klima geschaffen werden kann.

Die Erfindung wird im nachfolgenden anhand der in den Zeichnungen dargestellten Ausführungsbeispiele näher erläutert.

Es zeigen:
- Fig. 1: ein Blockschaltbild mit den Grundmodulen für eine Anlage der erfindungsgemäßen Art;
- Fig. 2: eine erfindungsgemäße Anlage, in Draufsicht;
- Fig. 3: die Dosisverteilung in bestrahlten Gütern in Abhängigkeit von der Eindringtiefe;
- Fig. 4: eine Ausführungsvariante einer erfindungsgemäßen Anlage, in Draufsicht;
- Fig. 5: eine Ausführungsvariante einer Elektronenaustrittsvorrichtung;
- Fig. 6: eine vereinfacht dargestellte Ausführungsvariante eines Teils des Fördersystems, in Seitenansicht;
- Fig. 7: eine weitere, vereinfacht dargestellte Ausführungsvariante des Prozeßförderers, in Draufsicht;
- Fig. 8a-8e: eine schematische Darstellung des zeitlichen Ablaufes des Transportes von Gütern im Bereich des Querförderers zur Erzielung einer mehrseitigen Bestrahlung der Güter.

Einführend sei festgehalten, daß in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale aus den gezeigten unterschiedlichen Ausführungsbeispielen für sich eigenständige, erfindungsgemäße Lösungen darstellen.

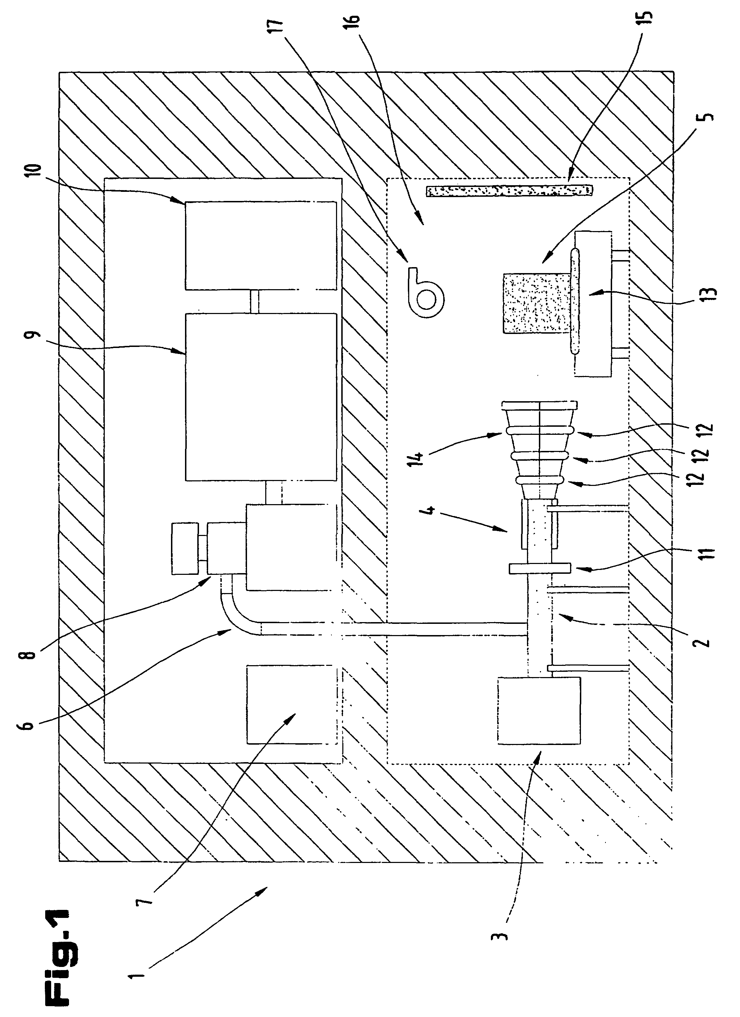

Fig. 1 zeigt die Grundmodule einer erfindungsgemäßen Anlage 1 schematisch als Blockschaltbild. In einem Linearbeschleuniger 2 werden von einer beheizten Glühkathode 3 Elektronen freigesetzt, die dann über die Länge des Linearbeschleunigers beschleunigt und mit Quadrupolmagneten 4 fokusiert werden. Anstelle des Linearbeschleunigers 2 können selbstverständlich alle anderen Arten von Beschleunigern, beispielsweise Ringbeschleuniger, verwendet werden.

Die erfindungsgemäße Anlage 1 stellt bevorzugt eine Beamleistung zwischen 5 kW und 30 kW, insbesondere 15 kW, zur Verfügung.

Die Energie der Elektronen, die zum Verändern der Eigenschaften von Gütern 5, beispielsweise dem Sterilisieren, benötigt wird, liegt vorzugsweise zwischen 5 MeV und 30 MeV, insbesondere 10 MeV. Da aufgrund dieser hohen Energien die Beschleunigung mit elektrostatischen Feldern, wie sie beispielsweise in herkömmlichen Bildröhren mit einer Energie von ca. 30 kV verwendet werden, nicht möglich ist, werden zur Beschleunigung der Elektronen vorzugsweise elektromagnetische Wellen verwendet.

Zur Erzeugung dieser elektromagnetischen Wellen entlang der Beschleunigungsstrecke benutzt man bevorzugt Hohlraumresonatoren 6, sogenannte Wave Guides. In diesen Hohlraumresonatoren 6, die beispielsweise aus Metallen wie Kupfer, Legierungen daraus wie Bronze, Messing, Stahl, Keramik oder aus Kunststoffen gearbeitet sein können, bildet sich eine stehende Welle aus, deren Feldverteilung durch die Geometrie der Hohlraumresonatoren 6 und der Frequenz der eingespeisten Mikrowellenenergie bestimmt wird.

Die stehende Welle in der Beschleunigereinheit wird von bevorzugt gepulsten Mikrowellen angeregt, die von einem Oszillator 7, einem sog. RF-Driver, erzeugt und durch einen Mikrowellenverstärker 8, z.B. einem Klystron, verstärkt werden. Die dazu notwendige elektrische Energie liefert ein Hochspannungsmodulator 9, der von einer primären Energiequelle 10, beispielsweise dem öffentlichen Stromnetz, versorgt wird.

Die Frequenz der gepulsten Mikrowellen liegt üblicherweise im GHz-Bereich, wohingegen die Pulsfrequenz der aus dem Linearbeschleuniger 2 austretenden Elektronen vorzugsweise bei 300 Pulsen pro Sekunde liegen kann.

Die derart beschleunigten und fokusierten Elektronen beaufschlagen, sofern ein Shutter 11 geöffnet ist, mit Hilfe von Ablenkmagneten 12 in einem definierten, vorbestimmbaren Verlauf die Güter 5. Die Güter 5 werden dabei, wie nachstehend noch näher erläutert wird, mit Hilfe eines Fördersystems 13 an einer Elektronenaustrittsvorrichtung 14, beispielsweise einem Scan-Horn, aus dem der Elektronenstrahl beispielsweise über ein Fenster aus einer Metallfolie austritt, vorbeibewegt. Die Dimensionen der Elektronenaustrittsvorrichtung 14 sind dabei vorzugsweise so gewählt, daß eine Scanhöhe bis zu 100 cm, bevorzugt 60 cm, erreicht werden kann. Somit ist es möglich, Güter 5 bis zur gewählten Scanhöhe mit dem Elektronenstrahl zu überstreichen. Für große, insbesondere sperrige Güter, deren Höhenabmessung außerhalb dieses Bereiches liegt, können die Dimensionen einer erfindungsgemäßen Anlage 1 durch Adaption an die entsprechenden Erfordernisse angepaßt werden. Um die erforderlichen Dimensionen zu erreichen, sind vor allem die Höhenabmessung der Elektronenaustrittsvorrichtung 14 sowie die dieser Elektronenaustrittsvorrichtung 14 zugeordneten Ablenkeinrichtungen für den Elektronenstrahl, beispielsweise elektrische Spulen, entsprechend zu verändern. Zur Sicherheit ist hinter den Gütern 5 in der Flucht des Linearbeschleunigers 2 ein Beam-Stop 15, beispielsweise eine Aluminiumplatte, vorzugsweise an einer Begrenzung eines Bestrahlungsraumes 16, angebracht, sodaß eventuell durchschlagende Elektronen abgebremst werden, wobei bevorzugt weiche γ-Strahlung entstehen kann. Da der Bestrahlungsraum 16 für Wartungsarbeiten von Menschen betreten werden können muß, ist zur Aufrechterhaltung eines verträglichen Klimas im Bestrahlungsraum 16 eine Entlüftungseinrichtung 17, beispielsweise ein Ventilator, vorgesehen.

Es sei an dieser Stelle erwähnt, daß selbstverständlich auch sämtliche anderen dem Stand der Technik entsprechenden Methoden zur Beschleunigung der Elektronen verwendet werden können. Vor allem ist die Ausbildung einer Anlage 1 nach der Erfindung nicht beschränkt auf die Verwendung von Mikrowellen als Beschleunigungsmedium, welche von einem Klystron verstärkt werden.

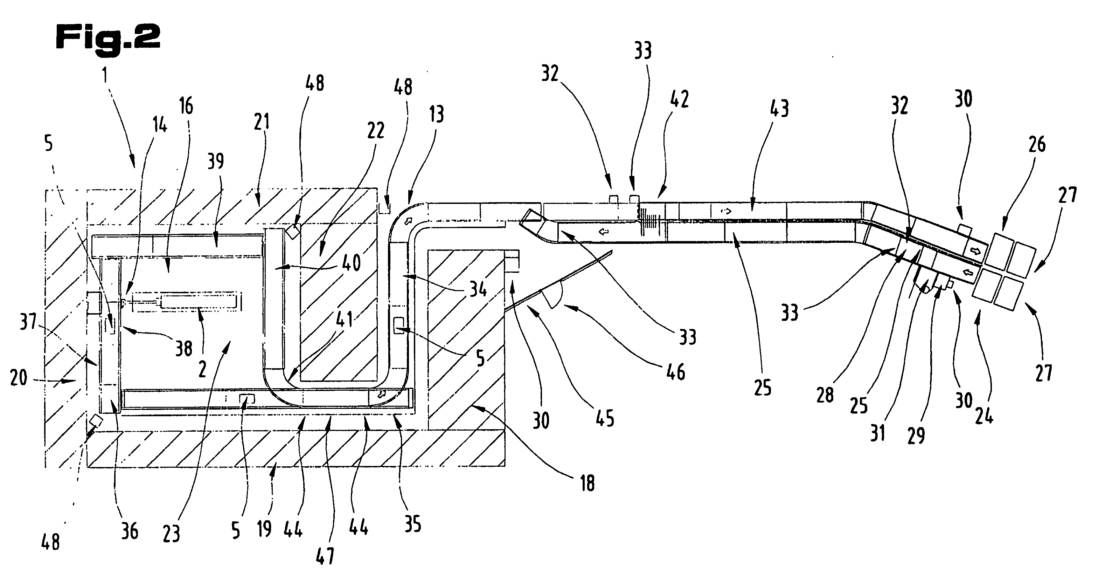

Fig. 2 zeigt die erfindungsgemäße Anlage schematisch vereinfacht in Draufsicht. Der Linearbeschleuniger 2 ist im Bestrahlungsraum 16 situiert, der von Wänden 18 bis 22 umgeben ist. Die Wände 18 bis 22 sind vorzugsweise so ausgeführt, daß sie ein Austreten der Elektronen bzw., da es sich hierbei um ionisierende Strahlung handelt, von Reaktionsprodukten der Atmosphäre im Bestrahlungsraum 16, beispielsweise Ozon, verhindern. Vorzugsweise bestehen die Wände 18 bis 22 ebenso wie eine Bodenplatte 23 und die in Fig. 2 nicht dargestellte Deckplatte, welche den unteren und oberen Abschluß des Bestrahlungsraumes 16 bilden, aus Stahlbeton. Denkbar sind jedoch auch andere Materialien, die voranstehend genannte Aufgaben erfüllen, z.B. Wände mit Verkleidungen aus Metallen mit hohem Elektroneneinfangquerschnitt. Die Dicke dieser Wände 18 bis 22 richtet sich nach den jeweiligen landesspezifischen Vorschriften bezüglich Strahlenschutz und sollte jedenfalls so ausgeführt sein, daß eine entsprechende Sicherheit der Anlage 1 gewährleistet werden kann. Vorzugsweise besitzt die Wand 20 eine Dicke von vier Metern und die Wände 19 und 21 eine Dicke von jeweils drei Metern. Die Wände 18 und 22 können dünner ausgeführt sein, sollten jedoch in Summe ebenfalls drei Meter ergeben. Die im Verhältnis zu den übrigen Wänden 18, 19, 21, 22 größere Breite der Wand 20 ergibt sich aus dem Umstand, daß der aus dem Linearbeschleuniger 2 austretende Elektronenstrahl auf diese Wand 20 gerichtet ist.

Wie aus Fig. 2 ersichtlich ist, werden die zu behandelnden Güter 5 mit dem Fördersystem 13 über einen labyrinthartigen Zugang dem Bestrahlungsraum 16 zugeführt und aus diesem wieder abtransportiert. Eine derartige Ausbildung des Zuganges zum Bestrahlungsraum 16 bietet den Vorteil, daß keine zusätzlichen Einrichtungen für die Zufuhr und den Abtransport der Güter 5, beispielsweise spezielle Schleusen, vorgesehen werden müssen, da ein Austreten von ionisierender Strahlung bzw. Reaktionsprodukten obengenannter Art weitestgehend verhindert wird. Prinzipiell ist zwar eine bestimmte Restdosis meßbar, jedoch liegt diese aufgrund des gewählten Designs der Schirmung unterhalb der von Gesetzes wegen vorgegebenen Grenzwerte. Zusätzlich sorgt die Entlüftungseinrichtung 17 (in Fig. 2 nicht dargestellt), wie bereits erwähnt, für eine dem Menschen ungefährliche Atmosphäre.

In Fig. 2 ist auch die bevorzugte Ausführungsvariante für das Fördersystem 13 dargestellt. Dieses besteht im wesentlichen vorzugsweise aus Stetigförderern, z.B. einer Rollenbahn, einem Stangenförderer, einem Kettenförderer, einem Rutschförderer, einem Bandförderer, oder dgl., und dient zur Beförderung von Gütern 5 verschiedener Größe. Bevorzugt ist das Fördersystem 13 in eine Aufgabebahn, eine Zuführbahn, eine Prozeßbahn, eine Staurollenbahn, einen Steigförderer, zumindest einen Querförderer, zumindest einem Eckumsetzer, eine Abnahmebahn sowie dem zugehörigen Equipment unterteilt. Denkbar ist jedoch auch, daß zumindest Teile des Fördersystems 13 aus anderen Beförderungseinrichtungen, beispielsweise endlose Gummibänder, Schwingförderer oder dgl., besteht. Das Fördersystem 13 kann bevorzugt zwei getrennte Bereiche umfassen, wobei ein unreiner Aufgabenbereich 24 durch eine Sperrvorrichtung 25, beispielsweise einem Sperrgitter, einer Trennwand, etc., vom reinen Abnahmebereich 26 getrennt ist.

Die Güter 5 können beispielsweise händisch von Beförderungseinheiten 27, beispielsweise Paletten, auf eine Aufgabebahn 28 gelegt werden, an der ein Bedienpult 29 mit einem Not-Aus-Schalter 30 plaziert ist. Die Güter 5 können von der in Bereichen angetriebenen Aufgabebahn 28 an einer Markierungsvorrichtung 31, beispielsweise einem Etikettenspender, einer Vorrichtung zum Anbringen von Mikrochips, einem Tintenstrahldrucker oder dgl., vorbeigeführt und dabei gleichzeitig gekennzeichnet werden. Zur automatischen Erfassung der Güter 5 und zur bevorzugten Verarbeitung der erfaßten Daten in einer EDV-Anlage ist an der Aufgabebahn 28 ein Scanner 32, beispielsweise ein Lesegerät, vor einem Gutstop 33 angebracht. Werden als Gutmarkierung Mikrochips verwendet, so ist es beispielsweise auch möglich, diese mit einem Sender, beispielsweise einem IR-Sender, auszustatten, von dem aus gutspezifische Daten einer Empfangseinheit, welche vorzugsweise im Bereich des Fördersystems 13 angebracht ist, übermittelt werden können.

Von der Rollenbahn der Aufgabebahn 28 bzw. dem Gutstopp 33 werden die Güter 5 an eine Zuführbahn 34 übergeben, welche bevorzugt ebenfalls als Rollenbahn ausgeführt ist. Die Zuführbahn 34 befördert die Güter 5 in den Bestrahlungsraum 16. An der Zuführbahn 34 kann vor jeder Richtungsänderung des Transportsystems ein Gutstop 33 angebracht sein, um eine möglichst lange Pufferstrecke zu erhalten. Der Gutstop 33 besteht bevorzugt aus einer Ventilspule, zwei Reedschaltern und einer Driverrolle. Denkbar sind natürlich auch andere Ausführungsvarianten, die beispielsweise Lichtschranken, mechanische Schalter, magnetische Impulsgeber, Videogeräte, oder dgl. umfassen.

Von der Rollenbahn der Zuführbahn 34 werden die Güter im Bestrahlungsraum 16 an einen Förderer 35 übergeben, der bevorzugt als Kettenförderer ausgebildet ist. Die Bevorzugung eines Kettenförderers trägt dem Umstand Rechnung, daß das Fördersystem 13 insgesamt über einen langen Zeitraum wartungsfrei sein soll, vor allem in Hinblick auf die Belastung durch ionisierende Strahlung.

In den Ecken des Bestrahlungsraumes 16 werden die Güter mit Eckumsetzern transportiert, welche ebenfalls wieder bevorzugt als Kettenförderer ausgebildet sind. Sobald der Schwerpunkt der Güter 5 über den Rollenmittelpunkt geschoben wird, wird das Gut 5 an einen etwas tiefer liegenden Querförderer 36 übergeben, beispielsweise einem Querförderkettenband, dessen Geschwindigkeit bevorzugt größer ist als die des Förderers 35. Bei diesem Vorgang dreht sich das Objekt um annähernd 90° um einen Eckanschlag (in Fig. 2 nicht dargestellt) und wird zugleich an der inneren Seitenbegrenzung der Kettenzuführung, beispielsweise einem Anschlag aus Metall, Kunststoff, oder dgl., ausgerichtet. Die Ausrichtung erfolgt dabei derart, daß die Güter 5, d.h. die zu bestrahlenden Oberflächen, in einem definierten Winkel zur Elektronenaustrittsvorrichtung 14, bevorzugt 90°, an dem Elektronenstrahl vorbeibewegt werden können. Selbstverständlich ist es aber auch möglich, die Güter 5 in jeder beliebigen relativen Lage zur Elektronenaustrittsvorrichtung 14 an dieser vorbeizubewegen, z.B. schief, quer, aufgestellt, etc.

Da alle Eckfördertechniken einen großen Abstand zwischen dem Transportgut erfordern und deshalb eine Optimierung des Wirkungsgrades der Anlage 1 verhindern, werden die Güter 5 vom Querförderer 36 auf einen Stauförderer 37, bevorzugt eine Staurollenbahn, übergeben, um so den Abstand zwischen den Gütern 5 soweit wie möglich zu verringern und damit eine wirtschaftliche Betreibung des Elektronenbeschleunigers zu ermöglichen. Der Stauförderer 37 besteht bevorzugt aus freidrehenden Rollen, die beidseitig an einer Kette montiert sind. Die Güter 5 werden dann bevorzugt lückenlos an einen Prozeßförderer 38 weitergegeben, der bevorzugt als Drahtgeflechtgurt ausgebildet ist und auf dem die Güter 5 an der Elektronenaustrittsvorrichtung 14 des Linearbeschleunigers 2 vorbeitransportiert werden. Die Geschwindigkeit der Übergabe der Güter 5 kann mit regelbaren Antriebseinrichtungen, z.B. Motoren, von der Steuerung so vorgegeben werden, daß beim Schließen der Lücken zwischen den Gütern 5 bereits auf dem Prozeßförderer 38 befindliche Güter 5 nicht verschoben werden.

Nach der Bestrahlung der Güter 5 werden diese mit einem Förderer 39, z.B. einem Kettenförderer, an einen Steigförderer 40, der beispielsweise ebenfalls als Kettenförderer ausgebildet sein kann, übergeben, von wo sie vorzugsweise über eine angetriebene Rollenbahn 41, welche bevorzugt über der Zuführbahn 34 angeordnet ist, aus dem Bestrahlungsraum 16 transportiert werden.

Im Bereich der Zuführung werden die Güter 5 bevorzugt auf zwei Etagen geführt, wobei die Zu- und die Abfuhr der Güter 5 sowohl in der oberen als auch in der unteren Etage wahlweise erfolgen kann. Nötigenfalls können diese beiden Teile des Fördersystems 13 aber auch nebeneinander geführt sein, um Güter 5 mit größeren Abmessungen zu transportieren. Die Führung in zwei Etagen bringt jedoch den Vorteil mit sich, daß die Dimension des labyrinthartigen Zuganges zum Bestrahlungsraum 16 relativ klein gehalten werden kann, womit eine erhöhte Sicherheit der Anlage 1 erreicht wird.

Im Bereich der Rollenbahn 41 kann eine Zählstation, beispielsweise ein Scanner 32 plaziert sein, mit dem der Bestrahlungsgrad der Güter 5 erkannt werden kann. Der Zählstation sind vorzugsweise drei Sensoren zugeordnet. Hinter dem Scanner 32 befindet sich wiederum ein Gutstop 33 vor einem Querförderer 42. Mit Hilfe dieses Querförderers 42 ist es möglich, die Güter 5 wieder auf die Aufgabebahn 28 zu überführen, sodaß diese um vorzugsweise 180° gedreht, dem Bestrahlungsprozeß erneut durchlaufen können.

Um jederzeit genau feststellen zu können, wie viele unbehandelte bzw. einseitig behandelte Güter 5 sich auf dem Fördersystem 13 befinden, können neben der Zählstation vor dem Querförderer 42 vorzugsweise zwei weitere Zählstationen mit bevorzugt je drei Sensoren zur gegenseitigen Kontrolle installiert sein. Bevorzugt befindet sich eine zusätzliche Zählstation in der Nähe der Elektronenaustrittsvorrichtung 14 sowie eine weitere Zählstation an der Aufgabebahn 28 im Bereich des Bedienpultes 29.

Den Abschluß des Fördersystems 13 bildet eine Abnahmebahn 43, mit der die Güter 5 aus dem Prozeß entfernt werden. Im Bereich der Abnahmebahn 43 kann ebenfalls aus Sicherheitsgründen ein Not-Aus-Schalter 30 angebracht sein.

Die Güter 5 können im Abnahmebereich 26 händisch von der Abnahmebahn 43 genommen und auf eine Beförderungseinheit 27, beispielsweise eine Palette, gegeben werden. Falls erforderlich, können die fertigbeladenen Beförderungseinheiten 27 für den Transport in einem Wickler mit einer Schrumpffolie versehen werden.

Der Antrieb einzelner Teile des Fördersystems 13 erfolgt vorzugsweise über Servomotoren. Denkbar sind jedoch auch alle anderen dem Stand der Technik entsprechenden Antriebsvorrichtungen. Eine Antriebsvorrichtung 44 des Transportsystems 13 sollte jedenfalls die Eigenschaft besitzen, daß das Gut 5 mit definierter, präziser Geschwindigkeit durch den Elektronenstrahl bewegt werden kann. Vorzugsweise werden Vortriebsgeschwindigkeiten im Bereich von 1 mm/s bis 400 mm/s, bevorzugt 5 mm/s bis 200 mm/s, verwendet, wobei die Vortriebsgeschwindigkeit auf die jeweils benötigte, zu verabreichende Dosis an Strahlung abzustimmen ist. Die Anzahl der zu verwendenden Antriebsvorrichtungen 44 sollte bevorzugt auf die Länge und die Anzahl an Einzelförderern abgestimmt werden.

Die Bestrahlung des Gutes 5 erfolgt vorzugsweise (wie dies aus Fig. 2 ersichtlich ist) horizontal, senkrecht auf die Transportrichtung des Gutes 5. Möglich ist jedoch auch, daß das Gut 5 von allen anderen Richtungen, wie beispielsweise von oben, von unten, etc., bestrahlt wird. Es können jedoch auch mehrere Beschleunigereinheiten verwendet werden, sodaß das Gut 5 gleichzeitig bzw. alternierend von mehreren Seiten bestrahlt werden kann. Denkbar ist auch, daß bei Verwendung von nur einer Beschleunigereinheit der Elektronenstrahl vor dem Austritt aus der Elektronenaustrittsvorrichtung 14 in zumindest zwei Teile gesplittet wird, beispielsweise durch Ablenkmagneten, und daß ein Teilstrahl alternierend zur Hauptstrahlrichtung, beispielsweise von oben, auf das Gut 5 trifft. Hierbei erweist sich der Einsatz eines Drahtgeflechtgurtes als Prozeßförderer 38 als besonders vorteilhaft, da auch bei erhöhter Strahlenbelastung durch Bestrahlung von oben dieser in der Regel über lange Zeitintervalle ohne Probleme eingesetzt werden kann.

Aufgrund der gewählten Beschleunigungsmethode kann zur Überstreichung der Oberfläche des Gutes 5 ein gepulster Elektronenstrahl verwendet werden. Dabei erweist es sich als vorteilhaft, mit mindestens 50%-iger Überlappung der Pulse zu arbeiten, wodurch eine homogene Dosisverteilung im Gut 5 erreicht wird. Dazu sollte natürlich die Vortriebsgeschwindigkeit des Fördersystems 7, im besonderen des Prozeßförderers 38, im Hinblick auf die zu verabreichende Dosis auch auf die Taktfrequenz des Elektronenstrahls abgestimmt werden.

Die maximal mögliche Vortriebsgeschwindigkeit des Prozeßförderers 38 wird insbesondere durch die Pulsrate, die Pulsdauer, die Scanfrequenz sowie die Anzahl der Pulse bestimmt.

Als besonders vorteilhaft erweist sich bei Verwendung einer erfindungsgemäßen Anlage 1 zur Bestrahlung von Gütern 5 im Vergleich zu den dem Stand der Technik entsprechenden Anlagen, daß die Güter 5 ohne Zwischenraum an der Elektronenaustrittsvorrichtung 14, beispielsweise dem Scan-Horn, vorbeitransportiert werden. Somit wird eine wesentliche Steigerung der Effizienz der Anlage 1 erreicht, da im Vergleich zu herkömmlichen Anlagen der Elektronenstrahl ständig auf die Oberfläche eines zu behandelnden Gutes 5 gerichtet ist. Zur weiteren Steigerung des Wirkungsgrades der Anlage 1 kann im Bereich des Prozeßförderers 38 ein Sensor, beispielsweise ein optischer Sensor, vorgesehen werden, der das Ende bzw. das Abreißen eines Gutstromes erkennt und diese Information an eine Steuereinrichtung für den Elektronenstrahlbeschleuniger übergibt, sodaß dieser beispielsweise in der Folge die Betriebsparameter der Beschleunigereinheit derart verändert, daß die Beschleunigereinheit im wesentlichen nur im stand-by-Betrieb geführt wird.

Der Shutter 11 erfüllt überwiegend die Aufgabe einer Schutzeinrichtung für die inneren, empfindlichen Teile des Elektronenbeschleunigers, um bei einem eventuellen Bruch bzw. einer Beschädigung des Elektronenaustrittsfensters im Bereich der Elektronenaustrittsvorrichtung 14 die genannten Teile des Elektronenbeschleunigers vor einer möglichen Beschädigung zu schützen.

Zur weiteren Erhöhung der Betriebssicherheit der erfindungsgemäßen Anlage 1 kann im Bereich des Eintritts des Fördersystems 13 in den labyrinthartigen Zugang zum Beschleunigungsraum 16 eine weitere Schutzeinrichtung 45 vorgesehen sein, welche beispielsweise aus einer Trennwand mit einer darin angebrachten Tür 46, einer entsprechenden Gitteranordnung oder dgl., bestehen kann. Somit ist ein unkontrollierter Zugang zum Bestrahlungsraum 16 in einem hohen Grad verwehrt. Zusätzlich können weitere Maßnahmen gesetzt werden, die einen derartigen unkontrollierten Zugang verhindern, beispielsweise können elektronische Verriegelungen der Schutzeinrichtung bzw. einer darin angebrachten Tür 46 den Zugang zum Bestrahlungsraum 16 verhindern, sofern zu diesem Zeitpunkt Elektronen aus der Elektronenaustrittsvorrichtung 14 austreten. An der Tür 46 kann bevorzugt ebenfalls ein Not-Aus-Schalter 30 angebracht sein.

Es ist jedoch auch möglich, die Steuerung der Beschleunigereinheit so auszubilden, daß über einen geeigneten Sensor, beispielsweise einen elektrischen Kontakt, einen optischen Sensor, oder dgl., im Rahmen der Tür 46 der Linearbeschleuniger 42 bei Öffnen der Tür 46 automatisch in den stand-by-Betrieb versetzt wird und somit eine potentielle Gefährdung von Personen durch austretende Elektronen beinahe zu 100 % auszuschließen ist. Als zusätzliche Sicherheitsmaßnahme kann entlang des Fördersystems 13 beispielsweise ein Seilzugschalter 47 vorgesehen werden. Dadurch kann es Personen ermöglicht werden, welche sich unbeabsichtigt bei Aktivierung der Beschleunigereinheit im Bestrahlungsraum 16 befinden, den Bestrahlungsprozeß zu unterbrechen bzw. bei entsprechender Auslegung einer Steuer- und Regeleinrichtung für die Beschleunigereinheit bei Ertönen eines optionalen akustischen Warnsignals der Steuerund Regeleinrichtung über den Seilzugschalter 47 mitzuteilen, daß der Elektronenbeschleuniger nicht in den Bestrahlungsbetrieb genommen werden darf. Weiters ist es möglich, elektrische Kontakte, beispielsweise in einer Trittmatte hinter der Tür 46, bzw. im labyrinthartigen Zugang, anzubringen, um auf diese Weise feststellen zu können, ob Personen den Bestrahlungsraum 16 betreten, während die Beschleunigereinheit im Betrieb ist. Dies setzt natürlich voraus, daß die Abmessungen der Trittmatte so gewählt werden, daß ein Übersteigen letzterer nicht möglich ist.

Selbstverständlich können aber auch an bestimmten Stellen in der Anlage 1, beispielsweise in der Nähe der Tür 46 Lichtschrankenanlagen angebracht sein, die den Zugang zum Bestrahlungsraum 16 überwachen.

Weitere übliche Sensoren und Warneinrichtungen, wie beispielsweise Bewegungssensoren, Drehspiegelleuchten, Hupen etc. können selbstverständlich ebenfalls an jedem beliebigen Punkt der Anlage 1 angebracht sein.

Als besonders vorteilhaft erweist sich auch, wenn an neuralgischen Punkten der Anlage 1 Überwachungskameras 48 angebracht sind, mit welchen beispielsweise der Bestrahlungsraum 16 sowie die einzelnen Teile des labyrinthartigen Zuganges sowie des Fördersystemes 13 überwacht werden können.

Die Übertragung sämtlicher, während des Betriebes des Elektronenbeschleunigers gewonnen Daten, insbesondere der von optischen Sensoren stammenden Daten, kann beispielsweise über Lichtleiter erfolgen, wodurch eventuell auftretende Störungen aufgrund einer Beeinflussung durch den Betrieb des Elektronenbeschleunigers verringert werden können.

Als zusätzliche Schutzmaßnahme kann im Bereich des Strahlenganges des Linearbeschleunigers 2 ein Strahlunterbrecher angeordnet sein, der bei eventuell auftretenden Störfällen beim Betrieb der Anlage 1 oder bei Betreten des Bestrahlunssraumes 16 den Strahlengang automatisch unterbricht und somit ein Austreten von Elektronen aus dem Linearbeschleuniger 2, insbesondere der Elektronenaustrittsvorrichtung 14, nicht möglich ist.

Vorzugsweise wird die im Prozeß entwickelte Abwärme über dem Stand der Technik entsprechende Wärmetauscher zurückgewonnen und dem Prozeß wieder zugeführt.

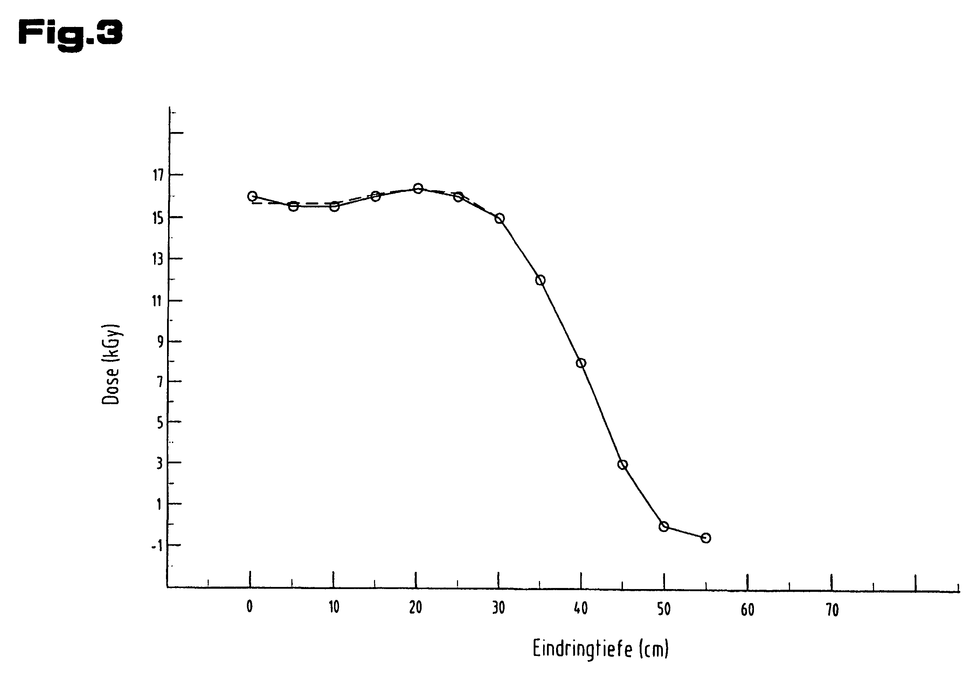

In Fig. 3 ist eine experimentell aufgenommene Reichweiteverteilung dargestellt, wobei auf der x-Achse die Eindringtiefe in das zu bestrahlende Gut 5 in Zentimetern und auf der y-Achse die übertragene Dosis in KGy aufgetragen ist. Zur Erstellung dieser Kurve wurde ein Testprodukt mit einer Dichte von 0,1 g/cm³ verwendet. Dieses wurde mit Elektronen mit einer Energie von 10 MeV und einer Strahlleistung von 15 kW bestrahlt.

Die Elektronen durchdringen die zu bestrahlenden, unter Umständen verpackten, Güter 5 und beginnen schon nach wenigen Zentimetern mit der zu bestrahlenden Materie wechselzuwirken. Dies wird nicht zuletzt bedingt durch den hohen Wirkungsquerschnitt der Elektronen und einer Dichte der zu bestrahlenden Güter 5 von ca. 1 g/cm³. Aus diesem Grund ist es meist notwendig, die Güter 5 doppelseitig der Bestrahlung auszusetzen. Darum ist das Fördersystem 13 auch vorzugsweise so ausgestaltet, daß bereits behandelte Güter 5 um bevorzugt 180° gedreht den Bestrahlungsprozeß erneut durchlaufen.

Typische Medikalprodukte haben üblicherweise eine Dichte von 0,05 g/cm³ bis 0,3 g/cm³ und können daher in ihrer Originalverpackung behandelt werden.

Aus Fig. 3 ist nun ersichtlich, daß in dem bestrahlten Testprodukt mit genannter Dichte bis zu einer Distanz von in etwa 30 cm die deponierte Dosis nahezu unabhängig von der Wegstrecke im Produkt ist. Somit ist es möglich, mit den gewählten Parametern Güter 5 bis zu einer Dicke von 60 cm beidseitig ohne Qualitätsverlust zu bestrahlen. Die Dicke des Produkts ist dabei auf die Fluchtlinie des Linearbeschleunigers 2 bezogen. Zur Bestrahlung von Gütern 5 mit größeren Abmessungen können die zu verwendenden Parameter entsprechend abgeändert werden.

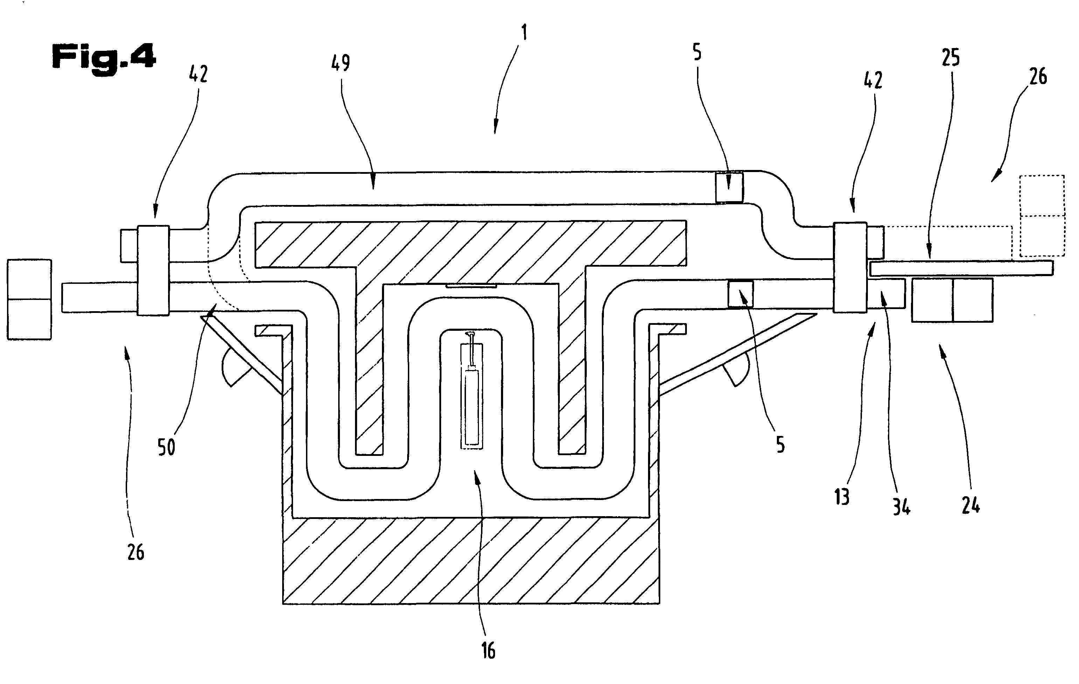

Fig. 4 zeigt schematisch eine weitere Ausführungsvariante einer erfindungsgemäßen Anlage 1 in Draufsicht. Im wesentlichen sind bei dieser Ausführungsvariante die einzelnen Komponenten die gleichen wie bei voranstehend beschriebener Ausführung. Der Unterschied besteht lediglich darin, daß für die Zufuhr und die Abfuhr der Güter 5 jeweils eigene, separate Labyrinthe verwendet werden. Dadurch wird es möglich, den unreinen Aufgabebereich 24 vom reinen Abnahmebereich 26 über den Bestrahlungsraum 16 zu trennen. In der Folge ist es damit auch möglich, speziell durch die Führung des Fördersystems 13 diese Anlage 1 auf einfache Art und Weise in einen Herstellungsprozeß für Güter 5 zu integrieren, wobei die halbfertigen Güter 5 vollautomatisch dem Aufgabebereich 24 (in Fig. 4 nicht dargestellt) zugeführt werden können, den Bestrahlungsprozeß durchlaufen und auf der gegenüberliegenden Seite im Abnahmebereich 26 abgenommen werden können. Natürlich ist dies bei der dargestellten Ausführungsvorrichtung nur möglich bei Gütern 5, deren Abmessungen und Dicke so gestaltet sind, daß eine einmalige Bestrahlung zur Erreichung der gewünschten Effekte ausreicht.

Ist dies jedoch nicht der Fall, so besteht auch hier wiederum die Möglichkeit, wie dies im oberen Teil der Fig. 4 gezeigt ist, über einen Querförderer 42 die Güter 5 nach dem erstmaligen Durchlaufen des Bestrahlungsprozesses an einen Rückförderer 49 zu übergeben. Der Rückförderer 49 kann, da seine Bestandteile zu keinem Zeitpunkt einer Bestrahlung ausgesetzt sind, aus einfachen und kostengünstigen Materialien, beispielsweise einem Endlosgummiband, aufgebaut sein. Sind im Rückförderer 49 mehrere Richtungsänderungen vorgesehen, wie dies in der Abbildung in Fig. 4 gezeigt ist, so kann dieser entsprechend unterteilt und aus dem Stand der Technik bekannten Elementen, beispielsweise Eckumsetzern, Rollenförderern mit 90°-Wendung, oder dgl. aufgebaut werden.

Wie dem rechten Teil der Fig. 4 zu entnehmen ist, werden die rückgeführten Güter 5 wiederum über einen Querförderer 42 der Zuführbahn 34 des Transportsystems 13 übergeben, um so den Bestrahlungsprozeß erneut zu durchlaufen.

Selbstverständlich besteht aber auch hier wiederum die Möglichkeit, anstelle eines zusätzlichen Querförderers 42 im linken Teil der Fig. 4 die Rückführung der Güter 5 über einen zusätzlichen Eckumsetzer 50 (in Fig. 4 strichliert dargestellt) dem Rückförderer 49 zuzuführen. In diesem Fall befindet sich der reine Abnahmebereich 26 auf der Seite des Bestrahlungsraumes 16, auf der auch der unreine Aufgabebereich 24 angeordnet ist. Die Trennung von unrein und rein kann dabei wieder über die bereits genannte Sperrvorrichtung 25 erfolgen.

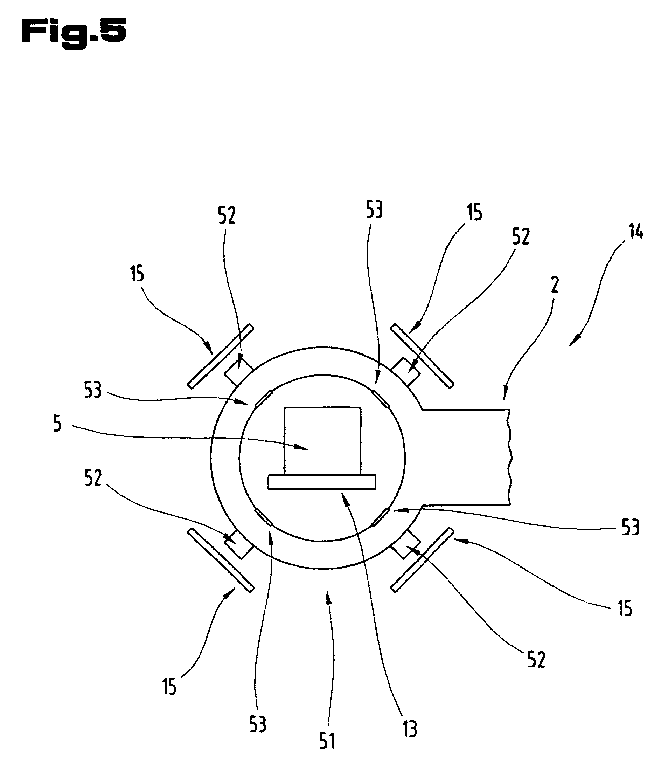

In Fig. 5 ist eine Variante für eine Elektronenaustrittsvorrichtung 14 schematisch dargestellt. Diese wird anstelle des Scan-Horns am vorderen Ende des Linearbeschleunigers 2 angebracht und besteht im wesentlichen aus einem Ring 51, auf dessen Umfang mehrere Ablenkvorrichtungen 52, beispielsweise Spulen, Magnete, oder dgl., angebracht sind. Dadurch wird erreicht, daß die Elektronen, welche sich auf einer Umlaufbahn innerhalb des Ringes 51 befinden, über Fenster 53, beispielsweise einer Metallfolie, die gegenüber den Ablenkvorrichtungen 52 plaziert sind, austreten und so in das zu bestrahlende Gut 5 eindringen können. Das zu bestrahlende Gut 5 wird dabei auf dem Fördersystem 13 durch den Ring 51 transportiert. Außerhalb des Ringes 51 sind oberhalb der Ablenkvorrichtungen 52 Beam-Stops 15 derart angebracht, daß eventuell durchschlagende Elektronen aus den gegenüberliegenden Fenstern abgefangen werden können.

Selbstverständlich ist es bei dieser Variante einer Elektronenaustrittsvorrichtung 14 möglich, beliebig viele Ablenkvorrichtungen 52, Fenster 53 und Beam-Stops 15 über den Umfang des Ringes 51 anzuordnen. Es ist dabei jedoch darauf zu achten, daß der Linearbeschleuniger nicht selbst durch eine unüberlegte Plazierung der Fenster 53 einer Bestrahlung unterliegt.

Mit einer erfindungsgemäßen Anlage 1 ist es vorteilhafterweise möglich, Güter 5 unterschiedlicher Art zu behandeln bzw. deren Eigenschaften in Teilbereichen zu verändern. So können beispielsweise Produkte unterschiedlichster Art sterilisiert werden. Dazu gehören unter anderem OP-Equipment, OP-Kleidung, Verbandsstoffe, OP-Abfall, Pharmarohstoffe, Pharmaverpackungen, Behältnisse aus Kunststoff und/oder Glas, Versuchsbehältnisse bzw. Versuchsequipment für den Biotechnologiebereich, die Sterilisation von Flüssigkeiten, Recyclingmaterialien und Müll im Bereich der Umwelttechnologie, die Entkeimung von Kunststoffen, die Sterilisation und Keimreduktion in Gewürzen, Rohstoffen, Produkten, Getränken, Verschlüssen sowie die Sterilisation von Verpackungen, Behältern oder Gefäßen für die Verpackungstechnologie.

Neben der Sterilisation gibt es aber eine Fülle von Einsatzmöglichkeiten für eine Anlage 1 der erfindungsgemäßen Art. Dazu gehört unter anderem die Veredelung von Oberflächen, beispielsweise durch Härten, die Härtung bzw. Vernetzung von Kunststoffen, die Aushärtung bzw. Vernetzung von Lacken. Bei Auswahl eines geeigneten Transportsystems, beispielsweise in Form von Rohrleitungen, können selbst Flüssigkeiten, z.B. Bier, Wasser oder dgl., und Gase ohne Zwischenverpackung auf diese Weise bestrahlt werden. Weiters können Schütt- und Stückgüter unterschiedlichster Art mit der erfindungsgemäßen Anlage 1 bestrahlt werden.

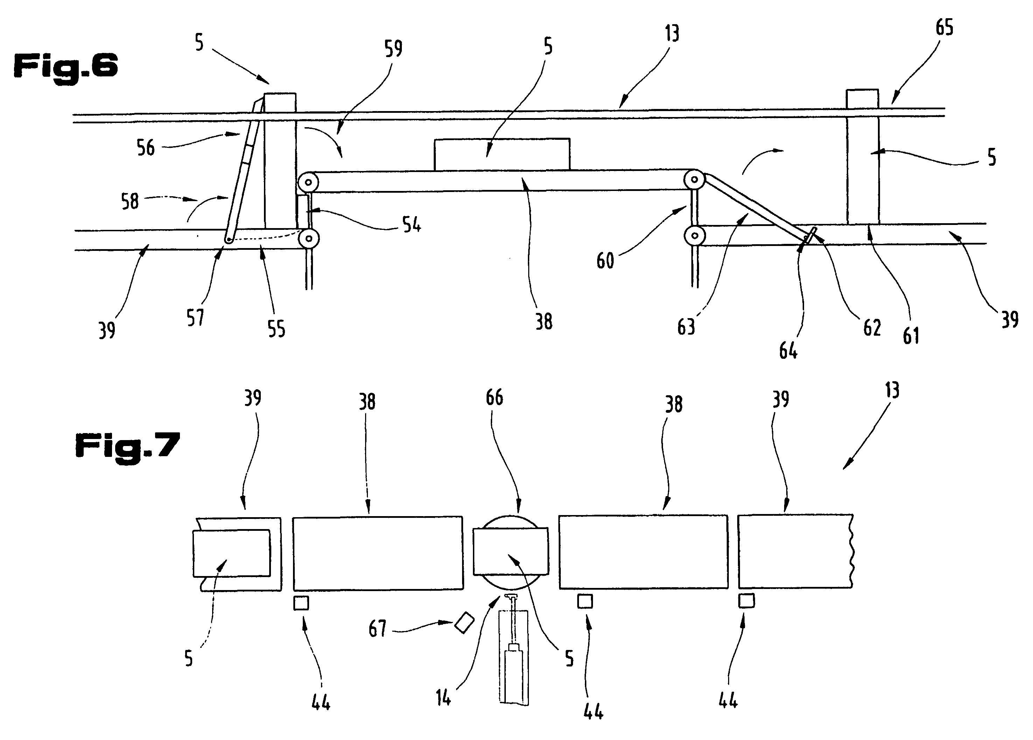

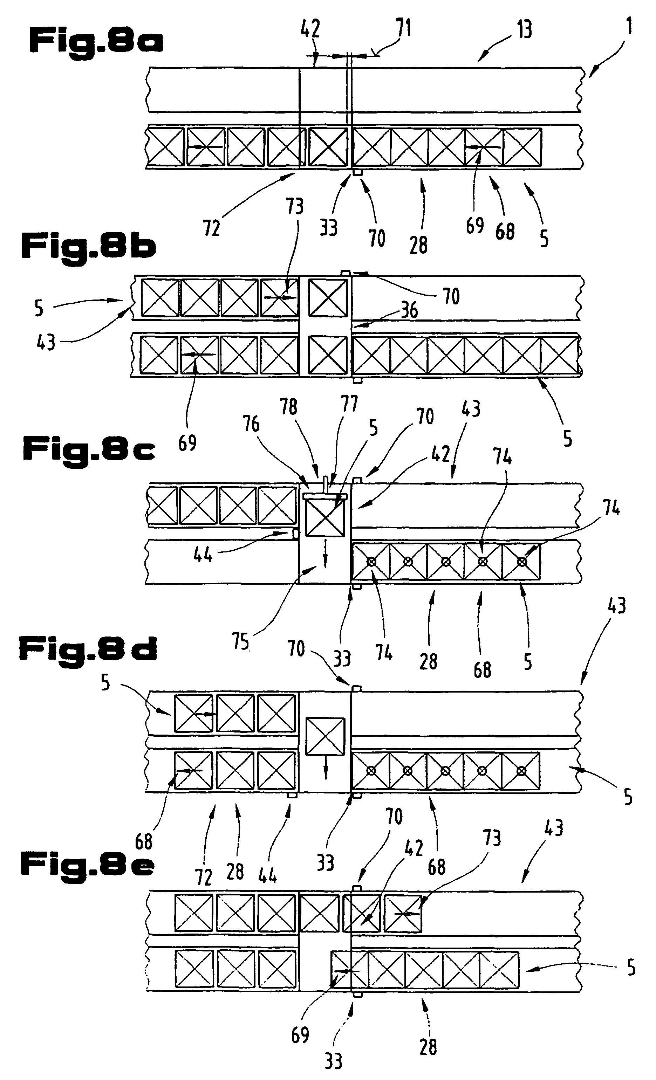

In Fig. 6 ist eine Ausführungsvariante für ein Fördersystem 13 gezeigt, mit welchem die Möglichkeit geschaffen wird, auch Langgüter in der erfindungsgemäßen Anlage 1 zu bestrahlen. Da es speziell bei Langgütern problematisch ist, diese in Bereichen mit 90°-Wendungen auf engstem Raum zu transportieren, wird mit einer Ausbildung gemäß der Fig. 6 eine Variante geschaffen, bei der die Langgüter bevorzugt stehend transportiert werden. Somit bestehen hinsichtlich der Länge der Güter 5 einzig die Beschränkungen, welche durch die Höhe des Labyrinths bzw. Bestrahlungsraumes entstehen. Da mit dieser Ausführungsvariante im wesentlichen die Teile des Fördersystems 13 identisch sind mit voranbeschriebenen Ausführungsvarianten, wird im folgenden nur jener Teil beschrieben, der die Unterschiede aufzeigt.

Aufgrund der Beschränkung der Bestrahlungshöhe (Scan-Höhe) durch die Elektronenaustrittsvorrichtung 14 (in Fig. 6 nicht gezeigt), beispielsweise dem Scan-Horn, ist es erforderlich, Güter 5, beispielsweise Langgüter, welche stehend durch den Bestrahlungsraum transportiert werden, vor der Elektronenaustrittsvorrichtung 14 in eine Position zu bringen, bei der die gesamte Oberfläche des Gutes 5 überstrichen werden kann. Es kann deshalb, wie im linken Teil der Fig. 6 gezeigt ist, auf der Zuführungsseite des Fördersystems 13 ein Kontakt 54 am Prozeßförderer 38 vorgesehen sein, beispielsweise ein elektrischer Kontakt, der auf Druck reagiert und der über eine Leitung 55 mit einer Betätigungseinrichtung 56, beispielsweise einem Hebel aus Metall, Kunststoff oder dgl. verbunden ist. Der Kontakt 54 kann zur Schonung des Gutes 5 beispielsweise mit einer Schaumstoffummantelung ausgerüstet sein.

Wird nun das Gut 5 mit dem Förderer 39 bis zum Kontakt 54 transportiert und übt auf diesen einen Druck aus, so wird über die Leitung 55 ein elektrisches Signal auf die Betätigungseinrichtung 56 übertragen, sodaß die Betätigungseinrichtung 56, die in einem Punkt 57 drehbar gelagert sein kann, in Richtung eines Pfeiles 58 nach oben bewegt wird, sodaß das Gut 5 gemäß Pfeil 59 eine Drehbewegung ausführt. Dadurch wird erreicht, daß das Gut 5 von seiner stehenden Position auf dem Förderer 39 in eine liegende Position auf dem Prozeßförderer 38 gebracht wird. In dieser liegenden Position wird das Gut 5 anschließend an der Elektronenaustrittsvorrichtung 14 (in Fig. 6 nicht dargestellt) vorbeibewegt und dabei gleichzeitig dessen auf die Elektronenaustrittsvorrichtung 14 weisende Oberfläche vom Elektronenstrahl überstrichen. Anschließend wird das Gut 5 über einen Kippunkt 60 von dem vorzugsweise höhergelegenen Prozeßförderer 38 auf den Förderer 39 gekippt, sobald sich der Schwerpunkt des Gutes 5 über den Kippunkt 60 hinausbewegt hat. Trifft nun eine Stirnfläche 61 des Gutes 5 auf einen Anschlag 62 einer Betätigungseinrichtung 63, welche in einem Punkt 64 drehbar gelagert sein kann, so wird das Gut 5 durch den Vorschub des Prozeßförderers 38 aufgerichtet und wieder in eine stehende Position gebracht, sodaß das Gut 5 wieder stehend aus dem Bestrahlungsraum transportiert werden kann. Die Betätigungseinrichtung 63 fällt automatisch in seine Ausgangsposition zurück, sobald der Anschlag 62 durch das Gut 5 freigegeben worden ist. Vorzugsweise wird diese dabei federnd abgefangen.

Um die seitliche Stabilität der Güter 5 zu gewährleisten, kann über die gesamte Länge des Fördersystemes 13 eine Führungsvorrichtung 65 angebracht sein. Diese besteht vorzugsweise aus zwei höhen- und/oder breitenverstellbaren Schienen, womit erreicht werden kann, daß Güter 5 unterschiedlicher Breite und Höhe durch den Bestrahlungsraum transportiert werden können.

Um bei einer zu geringen Aufstandsfläche der Langgüter ein Kippen in zum Förderer 39 paralleler Richtung zu verhindern, können sämtliche dem Stand der Technik entsprechende Maßnahmen getroffen werden.

Selbstverständlich ist es auch möglich, den Prozeßförderer 38 gegenüber dem Förderer 39 tieferliegend anzuordnen, wobei damit aber der Nachteil einer zusätzlichen Höheneinschränkung verbunden ist. Für diesen Fall müssen die Betätigungseinrichtungen 56, 63 entsprechend adaptiert werden. Insbesondere sollte die Betätigungseinrichtung 63 am Anfang des Prozeßförderers 38 (im linken Teil der Fig. 6) plaziert werden, um die nunmehr herabgleitenden Langgüter aufrichten zu können. Die Betätigungseinrichtung 56, in der Folge am Ende des Prozeßförderers situiert, kann anschließend an die Bestrahlung die Übergabe der Langgüter an den folgenden Förderer 39 übernehmen.

Die Förderer 39 und der Prozeßförderer 38 können - wie bereits erwähnt - als Stetigförderer ausgeführt sein.

Fig. 7 zeigt ein Fördersystem 13 in schematischer Darstellung und Draufsicht, mit dem es möglich ist, Güter 5 während eines Umlaufes mehrseitig zu bestrahlen. Dazu wird der Prozeßförderer 38, dem die Güter 5 durch den Förderer 39 zutransportiert werden, von einer Dreheinrichtung 66 unterbrochen. Diese Dreheinrichtung 66 ist vorzugsweise vor der Elektronenaustrittsvorrichtung 14 der Beschleunigereinheit plaziert. Werden nun Güter 5 vom Prozeßförderer 38 auf die Dreheinrichtung 66, die beispielsweise als Drehteller ausgebildet sein kann, befördert, so kann dies beispielsweise von einer Beobachtungseinrichtung 67, die z.B. als Sensor ausgebildet sein kann, erkannt werden. Dadurch wird der Förderprozeß unterbrochen und gleichzeitig eine Drehbewegung der Dreheinrichtung 66, bevorzugt um 180° bis 360° veranlaßt. Nach vollendeter Drehung, die beispielsweise über einen in Fig. 7 nicht dargestellten Sensor detektiert werden kann, werden die Antriebsvorrichtung 44 des Prozeßförderers 38 und in der Folge sämtliche anderen Antriebsvorrichtungen 44 des Fördersystems 13 mit Ausnahme der Dreheinrichtung 66 wieder aktiviert, sodaß ein nachfolgendes Gut 5 das auf der Dreheinrichtung 66 befindende Gut 5 in Richtung des zweiten Teils des Prozeßförderers 38 schiebt und letzteres dadurch über die weiteren Teile des Fördersystems 13 aus dem Bestrahlungsraum gebracht wird.

Um eine gegenseitige Behinderung der Güter 5 auf dem Prozeßförderer 38 und der Dreheinrichtung 66 zu verhindern, ist es beispielsweise möglich, die Dreheinrichtung 66 nach deren Beschickung mit einem Gut 5 aus der Ebene des Prozeßförderers 38 zu bewegen, z.B. anzuheben bzw. abzusenken. Außerdem können zum leichteren Transport von Gütern 5 auf der Dreheinrichtung 66 weitere Fördereinrichtungen, z.B. Rollenförderer mit oder ohne Antrieb oder dgl., vorhanden sein, sodaß ein Gut 5 automatisch auf der Dreheinrichtung 66 zentriert werden kann, sobald ein vorzugsweise installierter Sensor erkennt, daß ein Gut 5 auf die Dreheinrichtung 66 bewegt wird.

In den Fig. 8a bis 8e ist im Detail der Querförderer 42 des Fördersystems 13 der Anlage 1 schematisch dargestellt, um den Ablauf des Transportes von Gütern 5 in diesem Bereich bzw. den zeitlichen Ablauf des Transportes von Gütern 5 für eine mögliche zweiseitige Bestrahlung zu verdeutlichen.

Fig. 8a zeigt Güter 5 auf der Aufgabebahn 28, wobei die Güter 5 unverpackt bzw. in geeigneten Verpackungen, z.B. Kartons, transportiert werden. In einem Bereich 68 liegen die Güter 5 dabei dicht aufgeschlossen, d.h. ohne Zwischenraum vor und werden gemäß Pfeil 69 in Richtung des nicht dargestellten Bestrahlungsraums 16 dem ebenfalls nicht dargestellten Linearbeschleuniger 2 zugeführt. Ein Sensor 70, der beispielsweise als optischer Sensor, beispielsweise als Lichtschranke, als magnetischer Sensor oder dgl., ausgeführt sein kann, erkennt das Vorhandensein von Gütern 5 in seinem Bereich und aktiviert aufgrund der gewonnen Daten den Gutstop 33. Dieser Gutstop 33 kann beispielsweise als Schiene aus verschiedensten Werkstoffen, wie Metall, Kunststoff oder dgl., ausgeführt sein und hat eine Ruheposition unterhalb der Aufgabebahn 28 bzw. überragt nach Betätigung mit einem Teil seiner Oberfläche die Aufgabebahn 28, sodaß Güter 5 von einem Weitertransport abgehalten werden können. Der Gutstop 33 kann mit sämtlichen, dem Stand der Technik entsprechenden Betätigungseinrichtungen, wie beispielsweise pneumatische, elektrische, hydraulische, etc., betätigt werden. Vor der Betätigung des Gutstops 33 ist jedoch darauf zu achten, daß über eine Länge 71 auf der Aufgabebahn 28 keine Güter 5 vorhanden sind, um ein unbeabsichtigtes Abheben der Güter 5 von der Aufgabebahn 28 zu vermeiden. Um diese freie Länge 71 zu erreichen, werden die über den Bereich 68 zugeführten Güter 5 kurzzeitig gestoppt und in einem Bereich 72 vorhandene Güter 5 kontinuierlich weitertransportiert. Dies bringt zudem den Vorteil mit sich, daß zwischen den einzelnen Gütern 5 nunmehr der erforderliche Zwischenraum besteht und eine Eckförderung der Güter 5 möglich wird. Wie bereits beschrieben, wird dieser Zwischenraum in der Folge von dem in Fig. 8 nicht dargestellten Stauförderer 37 wieder so weit wie möglich reduziert, um eine optimale Ausnutzung des Linearbeschleunigers 2 zu erreichen.

Fig. 8b stellt nun den zeitlichen Ablauf des Transportes von doppelt zu bestrahlenden Gütern 5 zu einem Zeitpunkt dar, bei dem unbehandelte Güter 5 gemäß dem Pfeil 69 dem Bestrahlungsraum 16 zugeführt werden und bei dem bereits einfach, d.h. über eine Oberfläche bestrahlte Güter 5 den Bestrahlungsraum 16 über die Abnahmebahn 42 gemäß Pfeil 73 verlassen. Durch einen weiteren Sensor 70 im Bereich der Abnahmebahn 43 kann über eine nicht dargestellte Regel- und Steuereinrichtung, beispielsweise eine EDV-Anlage, das Vorhandensein von Gütern 5 auf der Abnahmebahn 43 im Bereich des Querförderers 36 erkannt werden.

In der Folge werden, wie in Fig. 8c dargestellt, Güter 5 im Bereich 68 der Aufgabebahn 28 vor dem Gutstop 33 gepuffert (in Fig. 8c durch Kreise 74 angedeutet) und wird das Gut 5, welches sich auf dem Querförderer 42 im Bereich der Abnahmebahn 43 befindet, gemäß Pfeil 75 in Richtung Aufgabebahn 28 bewegt. Diese Bewegung wird aufgrund der Daten vom im Bereich der Abnahmebahn 43 sich befindenden Sensor 70 initiiert und kann mit den unterschiedlichsten, dem Stand der Technik entsprechenden Bewegungstechniken erfolgen. Beispielsweise ist es möglich, das Gut 5 mit Hilfe einer Bewegungseinrichtung 76, die z.B. aus einem Schieber 77 und einem Gestänge 78 aufgebaut sein kann, auf den Bereich des Querförderers 42 der Aufgabebahn 28 zu verschieben. Diese Bewegungseinrichtung 76 kann aber auch pneumatisch ausgeführt sein, beispielsweise ist es möglich, das Gut 5 mit einem entsprechenden Luftstoß in besagte Richtung zu bewegen. Andererseits ist es aber natürlich auch möglich, den Querförderer 42 zumindest zum Teil als Rollenbahn auszuführen und genügt somit ein kurzer Impuls mit Hilfe einer entsprechenden Bewegungseinrichtung 76 auf das Gut 5, um die gewünschte Bewegung, vor allem die gewünschte Richtung, zu erzielen.

Selbstverständlich ist es aber auch möglich, daß zumindest Teile des Querförderers 42 mit Antriebsvorrichtungen 44, beispielsweise einem Servomotor, versehen sind, wie dies in Fig. 8c angedeutet ist.

Wie nun weiters in Fig. 8d dargestellt, werden die aus dem Bestrahlungsraum 16 kommenden Güter 5 erneut der Aufgabebahn 28 im Bereich 72 übergeben. Da die Aufgabebahn 28 im Bereich 72 vorzugsweise über eine Antriebsvorrichtung 44, beispielsweise einen Servomotor, angetrieben ist, werden die einfach bestrahlten Güter 5 gemäß Pfeil 69 erneut dem Bestrahlungsprozeß zugeführt, während dessen Güter 5 im Bereich 68 weiterhin gepuffert werden. Diese Pufferung der Güter 5 erfolgt dabei vorzugsweise so lange, bis das erste doppelt bestrahlte, d.h. über zwei gegenüberliegenden Oberflächen bestrahlte Gut 5 auf der Abnahmebahn 43 den entsprechenden, in diesem Bereich angeordneten Sensor 70 erreicht.

Als Folge davon wird über eine nicht dargestellte Steuer- und Regeleinrichtung, welche über den Sensor 70 aufgrund der an den Gütern 5 angebrachten Markierung, wie sie bereits voranstehend beschrieben worden ist, den Behandlungsgrad der Güter 5, also insbesondere auch doppelt bestrahlte Güter 5, erkennt, der Gutstop 33 in seine Ruheposition verbracht, sodaß kein Teil des Gutstops 33 über die Oberfläche der Aufgabebahn 28 hinausragt. Damit können nun, wie in Fig. 8e dargestellt, die gepufferten Güter 5 gemäß Pfeil 69 der Bestrahlung unterzogen werden, wohingegen fertig bestrahlte Güter 5 gemäß Pfeil 73 dem Bestrahlungsprozeß über die Abnahmebahn 43 entzogen werden.

Mit dem derart ausgebildeten Förderprozeß werden die Güter 5, da sie auf dem Querförderer 42 nicht gedreht werden sondern einzig eine Horizontalbewegung erfahren, auf der Aufgabebahn 28 so plaziert, daß sie den Bestrahlungsprozeß aufgrund des Rundumlaufes im Bestrahlungsraum 16 um annähern 180° um ihre Vertikalachse gedreht erneut durchlaufen können.

Es sei an dieser Stelle nochmals darauf hingewiesen, daß in den Fig. 8a bis 8e das Fördersystem 13 im Bereich des Querförderers 42 schematisch dargestellt ist und daß insbesondere an allen erforderlichen Positionen Antriebsvorrichtungen 44 bzw. Sensoren 70 angebracht sein können, um einen reibungslosen Ablauf der Beförderung der Güter 5 auf dem Fördersystem 13 zu ermöglichen.

Als Verfahren zur Bestrahlung von Gütern 5 hat sich die im folgenden beschriebene Vorgangsweise, die jedoch nicht zwingend ist und gegebenenfalls adaptiert werden muß, als vorteilhaft erwiesen.

Das zu bestrahlende Gut 5 wird im unreinen Aufgabebereich 24 dem Transportsystem 13 zugeführt. Dies kann beispielsweise händisch durch Herabnehmen der Güter 5 von Beförderungseinheiten 27, beispielsweise Paletten erfolgen.

Zur individuellen Kennzeichnung der Güter 5 werden diese an einer Markierungsvorrichtung 31, beispielsweise einem Etikettenspender, vorbeigeführt. Die derart angebrachte Markierung, welche z.B. aus einem Strich-Code bestehen kann, wird von einem nachfolgenden Scanner 32 erfaßt und diese Daten einer Steuer- und/oder Regeleinrichtung, beispielsweise einer EDV-Anlage zugeführt. Dem Scanner 32 ist ein erster Gutstop 33 zugeordnet, vor dem Güter 5 aus dem Aufgabebereich 24 gepuffert werden. Der Gutstop 33 gibt die Güter in aus dem Sterilisationsgrad genau errechneten Intervallen an die Zuführbahn 34 weiter.

Daraufhin wird das Gut 5 über die einzelnen Teile des Fördersystems 13 in den Bestrahlungsraum 16 verbracht. Für die im Gut 5 zu deponierende Dosis an Strahlenenergie ist es unter anderem wichtig, daß die Vortriebsgeschwindigkeit des Prozeßförderers 38 auf die Taktfrequenz des Linearbeschleunigers 2 abgestimmt wird. Da für die Beschleunigung der Elektronen gepulste elektromagnetische Wellen mit vordefinierter Frequenz, welche als stehende Wellen in Hohlraumresonatoren 6 vorliegen, die von einer gepulsten Mikrowelle, erzeugt von einem Oszillator 7, angeregt und von einem Klystron verstärkt werden, hat es sich als vorteilhaft erwiesen, wenn sich die Pulse der Elektronenstrahlung um mindestens 30 %, vorzugsweise 50 % überlappen. Auf diese Weise ist es möglich, im Gut 5 eine homogene Dosisverteilung zu erreichen.

Vorteilhaft ist eine Beschleunigeranlage, die eine Strahlenenergie im Bereich von 5 MeV bis 30 MeV, bevorzugt 10 MeV, und eine mittlere Beamleistung im Bereich von 5 kW bis 30 kW, vorzugsweise 15 kW, zur Verfügung stellt.

Die Vortriebsgeschwindigkeit des Prozeßförderers 38 sollte zwischen 1 mm/s und 400 mm/s, bevorzugt 5 mm/s und 200 mm/s betragen und allenfalls an die zu verabreichende Dosis angepaßt sein.

Die Bestrahlung des Gutes 5 erfolgt vorzugsweise horizontal und senkrecht zur Bewegungsrichtung des Gutes 5, kann aber auch von mehreren Seiten durchgeführt werden, vor allem dann, wenn mehrere Elektronenbeschleuniger eingesetzt werden. Im letzten Fall kann das Gut 5 alternierend oder simultan von mehreren Seiten bestrahlt werden, vorzugsweise in auf das Fördersystem 13 bezogener vertikaler bzw. horizontaler Richtung. Nach erfolgter Bestrahlung wird das Gut 5 über den labyrinthartigen Zugang wieder aus dem Bestrahlungsraum 16 transportiert und an einem Erkennungssystem, beispielsweise einem Scanner 32, insbesondere einem Lesegerät, vorbeigeführt. Die damit erfaßten Daten können ebenfalls einer EDV-Anlage zur Verfügung gestellt werden, welche auf diese Weise den Bestrahlungsgrad des Gutes 5 feststellen kann. Ist es erforderlich, daß das Gut 5 beidseitig bestrahlt wird, werden diese Daten einem Gutstop 33 zur Verfügung gestellt, der in Abhängigkeit vom Querförderer 42, Güter 5, welche sich auf der Zuführbahn 34 befinden, beispielsweise durch Ausfahren einer Metall- und/oder Kunststoffleiste vom Weitertransport in den Bestrahlungsraum 16 abhält. Dadurch wird es möglich, über den Querförderer 42 bereits bestrahlte Güter 5 erneut der Zuführbahn 34 und somit dem Bestrahlungsprozeß zuzuführen. Die Güter 5, die sich auf der Aufgabebahn 28 befinden, können von dem ersten Gutstop 33 solange aufgehalten und gepuffert werden, bis das erste doppeltbestrahlte Gut 5 wieder beim Querförderer 42 angelangt ist. Die Doppelbestrahlung wird wiederum über den Scanner 32 erfaßt.

Mit einer derartigen Steuerung wird auf vorteilhafte Weise eine sehr gute Auslastung der zur Verfügung stehenden Ressourcen erreicht. Fakultativ zu diesem ersten Gutstop 33 können über die Länge des Fördersystems 13 weitere Gutstops 33 angebracht sein. Vorzugsweise sind diese vor jeder Richtungsänderung des Transportsystems plaziert. Dadurch wird ein Puffer an Gütern 5 aufgebaut, sodaß in der Folge ein Abreißen des Gutstromes vor der Elektronenaustrittsvorrichtung 14 weitestgehend verhindert wird. Die Güter 5 werden von den jeweiligen Gutstops 33 in definierten Intervallen freigegeben, deren Zeitspanne sich nach der einzubringenden Strahlendosis richtet. In besonderen Fällen kann durch wiederholte Änderung der Bewegungsrichtung der Güter 5, d.h. von Teilen des Fördersystems 13, insbesondere des Prozeßförderers 38, diese zur Erreichung der notwendigen Strahlendosis im Gut 5 mehrmals am Elektronenstrahl vorbeigeführt werden.

Üblicherweise erfolgt der Zu- und Abtransport der Güter 5 zu und aus dem Bestrahlungsraum 16 auf einer Seite des Bestrahlungsraumes 16. Es ist jedoch auch möglich, die Zufuhr und den Abtransport der Güter 5 zu und aus dem Bestrahlungsraum 16 auf unterschiedlichen Seiten des Bestrahlungsraumes 16 vorzunehmen.

Zur Ermittlung der erforderlichen Dosis können vorab an einzelnen Gütern 5 Dosimeter, beispielsweise radiochrome Filmdosimeter, an signifikanten Stellen angebracht werden und diese Güter 5 dem Bestrahlungsprozeß ausgesetzt werden. Derartige signifikante Stellen sind beispielsweise jene Stellen, die sich durch erhöhte bzw. zu geringe Dosisübertragung auszeichnen. Dies ist vor allem im Inneren, aber auch an den Eckpunkten der Güter 5 zu erwarten, besonders dann, wenn die Güter 5 in einer Verpackung, beispielsweise ihrer Originalverpackung, bestrahlt werden, sodaß die zu verwendenden Dosimeter bevorzugt an diesen Stellen einzusetzen sind.

Radiochrome Filmdosimeter zeichnen sich dadurch aus, daß sie ihre Farbe in Abhängigkeit von der Bestrahlungsdauer bzw. der verabreichten Dosis ändern. Diese Farbänderung kann in der Folge über ein Spektralphotometer durch Messung der Abschwächung der Intensität eines hindurchtretenden Lichtstrahles bzw. in Reflexion gemessen werden. Aus den dabei ermittelten Werten läßt sich die exakte Vortriebsgeschwindigkeit des Prozeßförderers 38 bei konstanter Beam-Leistung errechnen. Für die Erstellung der Kalibrierungskurve zur Auswertung der Filme haben sich Alanin Transferdosimeter als vorteilhaft erwiesen. Selbstverständlich ist es aber auch möglich, sämtliche andere Methoden zur Kalibrierung zu verwenden.

Zusätzlich zu radiochromen Filmdosimetern können auch zur Erhöhung der Sicherheit weitere Dosimeter, vorzugsweise kalorimetrische Dosimeter eingesetzt werden.

Zur weiteren Sicherheitssteigerung können während des Produktionsprozesses in definierten Abständen weitere Filmdosimeter an den Gütern 5 angebracht werden, vorzugsweise Dosimeter mit derselben Markierung oder Kennung, wie sie für die Güter 5 verwendet werden. Auf diese Weise ist eine spätere unbeabsichtigte Verwechslung der einzelnen Filmdosimeter zu vermeiden. Diese mit den zusätzlichen Dosimetern versehenen Güter 5 können von einer Regel- und/oder Steuereinheit über beispielsweise den Scanner 32 erkannt werden.

Zur weiteren Nachbehandlung sowie zur Kontrolle der Stabilität des Prozesses können die aus den Dosimetern gewonnen Daten einer Datenverarbeitungsanlage zugeführt und von dieser archiviert werden.

Selbstverständlich ist es aber auch möglich, anhand der aus den Dosimetern gewonnen Daten in Art eines SOLL/IST-Vergleiches die Parametereinstellung der Beschleunigeranlage bzw. die Vortriebsgeschwindigkeiten der einzelnen Fördersystemteile zu regeln. Dazu können in der genannten Datenverarbeitungsanlage die SOLL-Werte hinterlegt sein.

Abschließend sei darauf hingewiesen, daß in den zuvor beschriebenen Ausführungsbeispielen einzelne Teile unproportional vergrößert dargestellt wurden, um das Verständnis der erfindungsgemäßen Lösung zu verbessern. Des weiteren können auch einzelne Teile der zuvor beschriebenen Merkmalskombination der einzelnen Ausführungsbeispiele in Verbindung mit anderen Einzelmerkmalen aus anderen Ausführungsbeispielen eigenständige, erfindungsgemäße Lösungen bilden.

Vor allem können die einzelnen in den Fig. 1, 2; 3; 4; 5; 6; 7; 8a bis 8e gezeigten Ausführungen den Gegenstand von eigenständigen, erfindungsgemäßen Lösungen bilden. Die diesbezüglichen erfindungsgemäßen Aufgaben und Lösungen sind den Detailbeschreibungen dieser Figuren zu entnehmen.

### Bezugszeichenaufstellung

- 1: Anlage
- 2: Linearbeschleuniger
- 3: Glühkathode
- 4: Quadrupolmagnet
- 5: Gut

- 6: Hohlraumresonator
- 7: Oszillator
- 8: Mikrowellenverstärker
- 9: Hochspannungsmodulator
- 10: Energiequelle

- 11: Shutter
- 12: Ablenkmagnet
- 13: Fördersystem
- 14: Elektronenaustrittsvorrichtung
- 15: Beam-Stop

- 16: Bestrahlungsraum
- 17: Entlüftungseinrichtung
- 18: Wand
- 19: Wand
- 20: Wand

- 21: Wand
- 22: Wand
- 23: Bodenplatte
- 24: Aufgabebereich
- 25: Sperrvorrichtung

- 26: Abnahmebereich
- 27: Beförderungseinheit
- 28: Aufgabebahn
- 29: Bedienpult
- 30: Not-Aus-Schalter

- 31: Markierungsvorrichtung
- 32: Scanner
- 33: Gutstop
- 34: Zuführbahn
- 35: Förderer

- 36: Querförderer
- 37: Stauförderer
- 38: Prozeßförderer
- 39: Förderer
- 40: Steigförderer

- 41: Rollenbahn
- 42: Querförderer
- 43: Abnahmebahn
- 44: Antriebsvorrichtung
- 45: Schutzeinrichtung

- 46: Tür
- 47: Seilzugschalter
- 48: Überwachungskamera
- 49: Rückförderer
- 50: Eckumsetzer

- 51: Ring
- 52: Ablenkvorrichtung
- 53: Fenster
- 54: Kontakt
- 55: Leitung

- 56: Betätigungseinrichtung
- 57: Punkt
- 58: Pfeil
- 59: Pfeil
- 60: Kippunkt

- 61: Stirnfläche
- 62: Anschlag
- 63: Betätigungseinrichtung
- 64: Punkt
- 65: Führungsvorrichtung

- 66: Dreheinrichtung
- 67: Beobachtungseinrichtung
- 68: Bereich
- 69: Pfeil
- 70: Sensor

- 71: Länge
- 72: Bereich
- 73: Pfeil
- 74: Kreis
- 75: Pfeil

- 76: Bewegungseinrichtung
- 77: Schieber
- 78: Gestänge

## Patentansprüche

1. Verfahren zum Verändern der Eigenschaften zumindest in einem Teilbereich und/oder zum Behandeln von Gütern (5), insbesondere eines Materials, eines Abfalls, eines Bauteils, eines Lebensmittels, einer Flüssigkeit, eines Gases, oder dgl., bei dem die Güter (5) mit einem Prozeßförderer (38) eines Fördersystem (13) an zumindest einer Elektronenbestrahlungseinrichtung, insbesondere einem Elektronenbeschleuniger, in einer Bestrahlungskammer vorbeitransportiert werden, wobei die zur Bestrahlung benötigten, aus einer Glühkathode austretenden Elektronen fokusiert und in einer Beschleunigereinheit mit Wellen einer bestimmten, vordefinierbaren Frequenz gepulst werden, mit einer bestimmten Frequenz aus der Bestrahlungseinrichtung austreten und auf die zu bestrahlenden Güter gelenkt werden, **dadurch gekennzeichnet, daß** die Güter dem Prozeßförderer (38) von einem Stauförderer (37) übergeben werden, wobei die Geschwindigkeit der Übergabe mit einer regelbaren Antriebseinrichtung von einer Steuerung so vorgegeben wird, daß bereits auf dem Prozeßförderer (38) befindliche Güter (5) nicht verschoben werden und die Güter (5) ohne Zwischenraum zueinander an der Elektronenbestrahlungseinrichtung vorbeitransportiert werden.

2. Verfahren nach Anspruch 1, **dadurch gekennzeichnet, daß** eine stehende Welle in der Beschleunigereinheit von einer gepulsten Mikrowelle angeregt wird.

3. Verfahren nach Anspruch 1 oder 2, **dadurch gekennzeichnet, daß** die Vortriebsgeschwindigkeit des Prozeßförderers (38), die Scanhöhe und die Anzahl der Pulse sowie die Pulsdauer so aufeinander abgestimmt werden, daß sich die Pulse der Elektronenstrahlung um mindestens 30 %, vorzugsweise 50 % überlappen.

4. Verfahren nach einem der vorhergehenden Ansprüche, **dadurch gekennzeichnet, daß** die Vortriebsgeschwindigkeit des Prozeßförderers (38) zwischen 1 mm/s und 400 mm/s, bevorzugt zwischen 5 mm/s und 200 mm/s beträgt.

5. Verfahren nach einem der vorhergehenden Ansprüche, **dadurch gekennzeichnet, daß** zur Bestrahlung des Gutes (5) mehrere Elektronenbeschleuniger eingesetzt werden.

6. Verfahren nach einem der vorhergehenden Ansprüche, **dadurch gekennzeichnet, daß** das Gut (5) alternierend oder simultan von mehreren Seiten bestrahlt wird, vorzugsweise in auf das Fördersystem (13) bezogener vertikaler bzw. horizontaler Richtung.

7. Verfahren nach einem der vorhergehenden Ansprüche, **dadurch gekennzeichnet, daß** zur Messung der in das Gut (5) eingebrachten Dosis Dosimeter, vorzugsweise radiochrome Filmdosimeter verwendet werden.

8. Verfahren nach Anspruch 7, **dadurch gekennzeichnet, daß** für die Erstellung einer Kalibrierungskurve zur Auswertung der Filme Alanin Transferdosimeter verwendet werden.

9. Verfahren nach Anspruch 7 oder 8, **dadurch gekennzeichnet, daß** nach der Auswertung der Filmdosimeter durch Messung der Intensität eines das Filmdosimeter durchdringenden Lichtstrahls in Transmission die erfaßten Kennwerte einer EDV-Anlage zur Nachbearbeitung, Speicherung und Kontrolle übergeben werden.

10. Verfahren nach einem der vorhergehenden Ansprüche, **dadurch gekennzeichnet, daß** die auf das Gut (5) einwirkende Strahlungsdosis über zusätzliche Dosimeter, vorzugsweise kalorimetrische Dosimeter, erfaßt wird.

11. Verfahren nach einem der vorhergehenden Ansprüche, **dadurch gekennzeichnet, daß** das Gut (5) mit einem Fördersystem (13) mit einer Geschwindigkeit an der Elektronenaustrittsstelle aus der Beschleunigereinheit, insbesondere dem Scan-Horn, vorbeitransportiert wird, die sich nach der zu verabreichenden Dosis richtet.

12. Verfahren nach einem der vorhergehenden Ansprüche, **dadurch gekennzeichnet, daß** an dem Gut (5) und/oder der Verpackung des Gutes zumindest eine Markierung, beispielsweise ein Strich-Code, angebracht wird, die von einem Erkennungssystem, beispielsweise einem Scanner (32), insbesondere einem Lesegerät, erkannt wird.

13. Verfahren nach einem der Ansprüche 7 bis 12, **dadurch gekennzeichnet, daß** an dem bevorzugt verwendeten Dosimeter, insbesondere dem Filmdosimeter zumindest eine Markierung zur nachträglichen Auswertung, Erkennung und Überprüfung des Dosimeters, insbesondere ein Strich-Code, angebracht wird, die von einem Erkennungssystem, beispielsweise einem Scanner, insbesondere einem Lesegerät, erkannt wird.

14. Verfahren nach Anspruch 13, **dadurch gekennzeichnet, daß** das Gut (5) nach dem Erkennungssystem an einem ersten Gutstopp (33) gepuffert wird.

15. Verfahren nach einem der vorhergehenden Ansprüche, **dadurch gekennzeichnet, daß** das Gut (5) bzw. die Güter (5) an zumindest zwei vorbestimmten Stellen des Fördersystems (13), insbesondere an den Stellen, an denen eine Änderung der Bewegungsrichtung stattfindet, gepuffert wird bzw. werden.

16. Verfahren nach einem der vorhergehenden Ansprüche, **dadurch gekennzeichnet, daß** das Gut (5) in aus dem Behandlungsgrad errechneten Intervallen einer Zuführfördereinrichtung zum Bestrahlungsraum (16) zugeführt werden.

17. Verfahren nach einem der vorhergehenden Ansprüche, **dadurch gekennzeichnet, daß** der Behandlungsgrad des Gutes (5) von einem Scanner (32), beispielsweise einem Strich-Code-Leser, oder dgl., erfaßt wird.

18. Verfahren nach einem der vorhergehenden Ansprüche, **dadurch gekennzeichnet, daß** das Gut (5) mehrmals am Elektronenstrahl vorbeigeführt wird, beispielsweise durch wiederholte Änderung der Bewegungsrichtung.

19. Verfahren nach einem der vorhergehenden Ansprüche, **dadurch gekennzeichnet, daß** das Gut (5) über eine Abnahmefördereinrichtung dem Prozeß entzogen wird.

20. Verfahren nach einem der vorhergehenden Ansprüche, **dadurch gekennzeichnet, daß** der Elektronenstrahl die Oberfläche des Gutes (5) in Form einer geometrischen Kurve überstreicht, die durch die Kombination der Vortriebsgeschwindigkeit mit der Bewegung des Elektronenstrahls festzulegen ist, insbesondere in Form einer Sägezahnkurve.

21. Verfahren nach einem der vorhergehenden Ansprüche, **dadurch gekennzeichnet, daß** an zumindest einem Gut (5) in einem Vorversuch die benötigte Strahlendosis mittels an markanten Stellen des Gutes (5), insbesondere an jenen Stellen, an denen die Strahlenbelastung wegen des Scan-Modus gering und/oder hoch ausfällt, beispielsweise in der Mitte der Oberfläche und/oder im Inneren des Gutes (5), in den Ecken des Gutes (5), angebrachten Dosimetern ermittelt wird.

22. Verfahren nach Anspruch 21, **dadurch gekennzeichnet, daß** die ermittelte benötigte Strahlendosis einer Steuereinheit, beispielsweise einer EDV-Anlage, zugeführt wird, welche mit diesen Werten die erforderlichen Geschwindigkeiten der einzelnen Fördereinrichtungen bestimmt und überwacht.

23. Verfahren nach einem der vorhergehenden Ansprüche, **dadurch gekennzeichnet, daß** zur Kontrolle während des Produktionsprozesses in definierten Intervallen an einzelnen Gütern (5), vorzugsweise an der äußeren Oberfläche, Dosimeter angebracht werden, mit deren Hilfe der Bestrahlungsprozeß kontrolliert werden kann.

24. Verfahren nach Anspruch 23, **dadurch gekennzeichnet, daß** ein der Steuereinheit zugeordnetes Erkennungssystem die mit Dosimetem versehenen Güter (5) erkennt.

25. Verfahren nach einem der vorhergehenden Ansprüche, **dadurch gekennzeichnet, daß** die aus dem Bestrahlungsprozeß einzelner Güter (5) gewonnen Daten einer Datenverarbeitung zugeführt werden.

26. Verfahren nach einem der vorhergehenden Ansprüche, **dadurch gekennzeichnet, daß** auf der Basis eines SOLL/IST-Vergleiches der eingebrachten Dosis die Geschwindigkeit einzelner Anlagenteile, insbesondere des Prozeßförderers (38), und/oder die Parametereinstellungen des Elektronenbeschleunigersystems geregelt wird.

27. Anlage (1) zum Verändern der Eigenschaften zumindest in einem Teilbereich und/oder zum Behandeln von Gütern (5) durch Strahlung, insbesondere eines Materials, eines Abfalls, eines Bauteils, eines Lebensmittels, einer Flüssigkeit, eines Gases, oder dgl., mit einem Prozeßförderer (38) eines Fördersystems (13) für die zu behandelnden Güter (5), mit einer Quelle zur Erzeugung freier Elektronen, mit einem Beschleunigersystem für die freien Elektronen, mit einem Bestrahlungsraum (16), in dem die Güter (5) bestrahlt werden und der von Strahlenschutzwänden umgeben ist, **dadurch gekennzeichnet, daß** das Fördersystem (13) derart ausgestaltet ist, daß die zu bestrahlenden Güter (5) zur Effizienzsteigerung der Anlage (1) ohne Zwischenraum zueinander an der Elektronenaustrittsvorrichtung (14) aus der Beschleunigereinheit vorbeitransportiert werden können, wobei in Förderrichtung vor dem Prozeßförderer (38) ein Stauförderer (37) mit einer von einer Steuerung geregelten Antriebseinrichtung angeordnet ist.

28. Anlage nach Anspruch 27, **dadurch gekennzeichnet, daß** die Zu- und Abfuhr der Güter (5) in und aus dem Bestrahlungsraum (16) an verschiedenen Seiten der Strahlenschutzwände durch jeweils eigene labyrinthartige Zugänge für die Zu- und Abfuhr der Güter (5) erfolgt.

29. Anlage nach Anspruch 27 oder 28, **dadurch gekennzeichnet, daß** zumindest an einer Stelle des Fördersystems (13) eine Einrichtung vorhanden ist, beispielsweise ein Anschlag aus einem Metall und/oder Kunststoff, oder dgl., sodaß eine automatische Ausrichtung der Güter (5) so erfolgt, daß diese in einem definierten Winkel zur Elektronenaustrittsvorrichtung (14), bevorzugt 90°, an dem Elektronenstahl vorbeitransportiert werden können.

30. Anlage nach einem der Ansprüche 27 bis 29, **dadurch gekennzeichnet, daß** das Fördersystem (13) außerhalb des Gefahrenbereiches des Elektronenbeschleunigers aus zwei getrennten Bereichen besteht, wobei der unreine Aufgabebereich (24) durch eine Sperrvorrichtung (25), z.B. ein Sperrgitter, eine Sperrwand, vom reinen Abnahmebereich (26) getrennt ist.

31. Anlage nach einem der Ansprüche 27 bis 30, **dadurch gekennzeichnet, daß** der Beschleuniger ein Ringbeschleuniger ist.

32. Anlage nach einem der Ansprüche 27 bis 31, **dadurch gekennzeichnet, daß** die Elektronenaustrittsvorrichtung (14) ein Scan-Horn mit einer Scanhöhe bis 100 cm, bevorzugt 60 cm, ist.

33. Anlage nach einem der Ansprüche 27 bis 31, **dadurch gekennzeichnet, daß** die Elektronenaustrittsvorrichtung (14) in Form eines Kreises um das Fördersystem (13) geführt ist.

34. Anlage nach einem der Ansprüche 27 bis 33, **dadurch gekennzeichnet, daß** der Elektronenbeschleuniger so ausgebildet ist, daß die im Gut (5) deponierte Dosis bis zu einer Eindringtiefe des Elektronenstrahls von 50 cm, bevorzugt 30 cm, weitgehend unabhängig von der Wegstrecke im Gut (5) ist.

35. Anlage nach einem der Ansprüche 27 bis 34, **dadurch gekennzeichnet, daß** am Fördersystem (13) zur Erreichung einer homogenen Dosisverteilung im Gut (5) eine Vortriebsgeschwindigkeit mit mindestens 30 %, vorzugsweise 50 %, Überlappung der Pulse einstellbar ist.

36. Anlage nach einem der Ansprüche 27 bis 35, **dadurch gekennzeichnet, daß** die Beschleunigereinheit so angeordnet ist, daß die Bestrahlung von oben und/oder unten erfolgt.

37. Anlage nach einem der Ansprüche 27 bis 36, **dadurch gekennzeichnet, daß** das Fördersystem (13) derart ausgebildet ist, daß eine gleichzeitige Bestrahlung des Gutes (5) von mehreren Seiten möglich ist.

38. Anlage nach einem der Ansprüche 27 bis 37, **dadurch gekennzeichnet, daß** am Fördersystem (13) eine Vortriebsgeschwindigkeit von 1 mm/s bis 400 mm/s, bevorzugt 5 mm/s bis 200 mm/s, eingestellt werden kann.

39. Anlage nach einem der Ansprüche 27 bis 38, **dadurch gekennzeichnet, daß** das Fördersystem (13) aus Stetigförderern, beispielsweise einer Rollenbahn und/oder einem Kettenförderer, besteht.

40. Anlage nach einem der Ansprüche 27 bis 39, **dadurch gekennzeichnet, daß** das Fördersystem (13) aus einer Aufgabebahn (28), einer Zuführbahn (34), einem Prozeßförderer (38), einem Stauförderer (37), einem Steigförderer (40), einem Querförderer (36, 42), zumindest einem Eckumsetzer und einer Abnahmebahn (43) besteht.

41. Anlage nach einem der Ansprüche 27 bis 40, **dadurch gekennzeichnet, daß** am Fördersystem (13) zumindest eine Markierungsvorrichtung (31) für das Gut (5), beispielsweise ein Etikettenspender, eine Vorrichtung zum Anbringen von Mikrochips, oder dgl., angeordnet ist.

42. Anlage nach Anspruch 41, **dadurch gekennzeichnet, daß** die Etiketten mit einem Strich-Code versehen sind.

43. Anlage nach Anspruch 41, **dadurch gekennzeichnet, daß** die verwendeten Mikrochips mit Sendeanlagen, beispielsweise einem IR-Sender, ausgerüstet sind.

44. Anlage nach Anspruch 43, **dadurch gekennzeichnet, daß** im Bereich des Fördersystems (13) eine Empfangsstation für IR-Strahlen angeordnet ist.

45. Anlage nach einem der Ansprüche 27 bis 44, **dadurch gekennzeichnet, daß** am Fördersystem (13) zumindest ein Scanner (32), beispielsweise ein Lesegerät, oder dgl., zur Guterfassung angebracht ist.

46. Anlage nach einem der Ansprüche 27 bis 45, **dadurch gekennzeichnet, daß** das Fördersystem (13) zumindest einen Gutstop (33) umfaßt.

47. Anlage nach Anspruch 46, **dadurch gekennzeichnet, daß** vor jeder Richtungsänderung des Fördersystems (13) ein Gutstop (33) angebracht ist.

48. Anlage nach Anspruch 46 oder 47, **dadurch gekennzeichnet, daß** die Betätigungseinrichtung für einen Gutstop (33) vorzugsweise aus einer Ventilspule, zwei Reedschaltern und einer Driverrolle besteht.

49. Anlage nach einem der Ansprüche 27 bis 48, **dadurch gekennzeichnet, daß** das Fördersystem (13) eine Zählstation zur Ermittlung der einseitig bestrahlten Güter (5) umfaßt.

50. Anlage nach Anspruch 49, **dadurch gekennzeichnet, daß** die Zählstation vorzugsweise drei Sensoren umfaßt.

51. Anlage nach einem der Ansprüche 27 bis 50, **dadurch gekennzeichnet, daß** der Prozeßförderer (38) vorzugsweise als Drahtgeflechtgurt ausgebildet ist.

52. Anlage nach einem der Ansprüche 27 bis 51, **dadurch gekennzeichnet, daß** die Anlage (1) zumindest einen Wärmetauscher umfaßt.

53. Anlage nach einem der Ansprüche 27 bis 52, **dadurch gekennzeichnet, daß** im Bestrahlungsraum (16) zumindest eine Entlüftungseinrichtung (17), beispielsweise ein Ventilator, angeordnet ist.

## Claims

1. Method of changing the properties of at least a part region of and/or for treating items (5), in particular a material, a waste product, a component, a foodstuff, a liquid, a gas or similar, whereby the items (5) are fed by means of a process conveyor (38) of a conveyor system (13) past at least one electron radiation facility, in particular an electron accelerator, in an irradiation chamber, the electrons needed for irradiation purposes being emitted from a glow cathode and focussed, pulsed through an accelerator unit with waves at a specific, pre-definable frequency, and emitted from the radiation facility at a specific frequency and directed onto the items to be irradiated, **characterised in that** the items are transferred to the process conveyor (38) from a buffer conveyor (37), the speed of the transfer being pre-set by a controllable drive mechanism of a controller so that items (5) which are already disposed on the process conveyor (38) are not moved and the items (5) are fed past the electron radiation facility without any spaces between them.

2. Method as claimed in claim 1, **characterised in that** a stationary wave in the accelerator unit is energised by a pulsed microwave.

3. Method as claimed in claim 1 or 2, **characterised in that** the feed rate of the process conveyor (38), the scanning height and the number of pulses and pulse duration are set relative to one another so that the pulses of the electron beam overlap by at least 30 %, preferably 50 %.

4. Method as claimed in one of the preceding claims, **characterised in that** the feed rate of the process conveyor (38) is between 1 mm/s and 400 mm/s, preferably between 5 mm/s and 200 mm/s.

5. Method as claimed in one of the preceding claims, **characterised in that** several electron accelerators are used to irradiate the item (5).

6. Method as claimed in one of the preceding claims, **characterised in that** the item (5) is irradiated from several sides alternately or simultaneously, preferably in the vertical and horizontal direction by reference to the conveyor system (13).

7. Method as claimed in one of the preceding claims, **characterised in that** film dosimeters, preferably of the radio-chromic type, are used to measure the dose introduced into the item (5).

8. Method as claimed in claim 7, **characterised in that** alanine transfer dosimeters are used to set up a calibration curve for evaluating the films.

9. Method as claimed in claim 7 or 8, **characterised in that**, following the evaluation by the film dosimeter obtained by measuring the intensity of a light beam passing through the film dosimeter in transmission, the detected characteristic values are transferred to an EDP unit for subsequent processing, storage and control.

10. Method as claimed in one of the preceding claims, **characterised in that** the radiation dose acting on the item (5) is detected by additional dosimeters, preferably calorimetric dosimeters.

11. Method as claimed in one of the preceding claims, **characterised in that** the item (5) is fed past the point at which the electrons are discharged from the accelerator unit, in particular the scan horn, by a conveyor system (13) at a speed which depends on the dose to be administered.

12. Method as claimed in one of the preceding claims, **characterised in that** at least one marking, for example a bar code, is applied to the item (5) and/or the packaging of the item, which is detected by a detection system, for example a scanner (32), in particular a reading device.

13. Method as claimed in one of claims 7 to 12, **characterised in that** at least one marking, in particular a bar code, is applied to the preferred dosimeter, in particular a film dosimeter, for subsequent evaluation, identification and checking of the dosimeter, which is detected by a detection system, for example a scanner, in particular a reading device.

14. Method as claimed in claim 13, **characterised in that** the item (5) is buffered at a first item stop (33) downstream of the detection system.

15. Method as claimed in one of the preceding claims, **characterised in that** the item (5) or items (5) is or are buffered at at least two predefined points of the conveyor system (13), in particular at the points at which the feed direction is changed.

16. Method as claimed in one of the preceding claims, **characterised in that** the item (5) is delivered to a delivery conveyor system leading to the irradiation chamber (16) at intervals calculated on the basis of the degree of treatment.

17. Method as claimed in one of the preceding claims, **characterised in that** the degree of treatment of the item (5) is detected by a scanner (32), for example a bar code reader or similar.

18. Method as claimed in one of the preceding claims, **characterised in that** the item (5) is fed past the electron beam several times, for example by repeatedly changing the direction of displacement.

19. Method as claimed in one of the preceding claims, **characterised in that** the item (5) is withdrawn from the process by means of a discharge conveyor system.

20. Method as claimed in one of the preceding claims, **characterised in that** the electron beam passes over the surface of the item (5) in the form of a geometric curve, in particular in the form of a sawtooth curve, which is determined by the combination of the feed rate and the motion of the electron beam.

21. Method as claimed in one of the preceding claims, **characterised in that** the required dose of irradiation is determined for at least one item (5) during a preliminary test by means of dosimeters attached to prominent points of the item (5), in particular to those points at which the irradiation applied is low and/or high due to the scanning mode, for example at the centre of the surface and/or in the interior of the item (5), in the comers of item (5).

22. Method as claimed in claim 21, **characterised in that**, once determined, the required irradiation dose is forwarded to a control unit, for example an EDP system, which uses these values as a means of determining and monitoring the requisite speeds of the individual conveyor systems.

23. Method as claimed in one of the preceding claims, **characterised in that** dosimeters are attached to individual items (5), preferably to the external surface, for control purposes at defined intervals during the production process, by means of which the irradiation process can be controlled.

24. Method as claimed in claim 23, **characterised in that** a detection system cooperating with the control unit detects the items (5) to which dosimeters are attached.

25. Method as claimed in one of the preceding claims, **characterised in that** the data obtained during the process of irradiating individual items (5) is forwarded to a data processing system.

26. Method as claimed in one of the preceding claims, **characterised in that** the speed of individual parts of the system, in particular the process conveyor (38), and/or the parameter settings for the electron accelerator system are controlled on the basis of a DESIRED/ACTUAL comparison of the applied dose.

27. Facility (1) for changing the properties in at least a part region of and/or for treating items (5) by irradiation, in particular a material, a waste product, a component, a foodstuff, a liquid, a gas or similar, incorporating a process conveyor (38) of a conveyor system (13) for the items (5) to be treated, a source for generating free electrons, an accelerator system for the free electrons, an irradiation chamber (16) in which the items (5) are irradiated and which is surrounded by radiation protection walls, **characterised in that** the conveyor system (13) is configured so that the items (5) to be irradiated can be fed past the device (14) emitting the electrons from the accelerator unit without any spaces between them in order to increase the efficiency of the facility (1), a buffer conveyor (37) with a drive system controlled by a controller being disposed upstream of the process conveyor (38) in the conveying direction.

28. Facility as claimed in claim 27, **characterised in that** the items (5) are fed into and out of the irradiation chamber (16) along different sides of the radiation protection walls by means of respective separate labyrinthine access points for admitting and discharging the items (5).

29. Facility as claimed in claim 27 or 28, **characterised in that** a device is disposed at at least one point of the conveyor system (13), for example a stop made from metal and/or plastic or similar, so that the items (5) are automatically oriented in such a way that they can be fed past the electron beam at a defined angle relative to the electron emitting device (14), preferably 90°.

30. Facility as claimed in one of claims 27 to 29, **characterised in that**, outside the feed-past zone of the electron accelerator, the conveyor system (13) consists of two separate regions and the unclean feeder region (24) is separated from the clean discharge region (26) by a barrier system (25), e.g. a barrier grate, a barrier wall.

31. Facility as claimed in one of claims 27 to 30, **characterised in that** the accelerator is a ring accelerator.

32. Facility as claimed in one of claims 27 to 31, **characterised in that** the electron emitting device (14) is a scan horn with a scanning height of up to 100 cm, preferably 60 cm.

33. Facility as claimed in one of claims 27 to 31, **characterised in that** the electron emitting device (14) is guided in a circle around the conveyor system (13).

34. Facility as claimed in one of claims 27 to 33, **characterised in that** the electron accelerator is designed so that the dose deposited in the item (5) is substantially independent of the travel path in the item (5) up to a penetration depth of the electron beam of 50 cm, preferably 30 cm.

35. Facility as claimed in one of claims 27 to 34, **characterised in that**, in order to obtain a homogeneous dose distribution in the item (5), a feed rate can be set on the conveyor system (13) so that there is an overlap of at least 30 %, preferably 50 %, of the pulses.

36. Facility as claimed in one of claims 27 to 35, **characterised in that** the accelerator unit is disposed so that the irradiation is directed from above and/or underneath.

37. Facility as claimed in one of claims 27 to 36, **characterised in that** the conveyor system (13) is designed so that an item (5) can be irradiated from more than one side simultaneously.

38. Facility as claimed in one of claims 27 to 37, **characterised in that** a feed rate of 1 mm/s to 400 mm/s, preferably 5 mm/s to 200 mm/s, can be set on the conveyor system (13).

39. Facility as claimed in one of claims 27 to 38, **characterised in that** the conveyor system (13) consists of continuous conveyors, for example a roller track and/or a chain conveyor.

40. Facility as claimed in one of claims 27 to 39, **characterised in that** the conveyor system (13) consists of a feeder track (28), a delivery track (34), a process conveyor (38), a buffer conveyor (37), an incline conveyor (40), a transverse conveyor (36, 42), at least one comer unit and a discharge track (43).

41. Facility as claimed in one of claims 27 to 40, **characterised in that** the conveyor system (13) has at least one marker device (31) for the item (5), for example a label dispenser, a device for attaching microchips or similar.

42. Facility as claimed in claim 41, **characterised in that** the labels are provided with a bar code.

43. Facility as claimed in claim 41, **characterised in that** the microchips used are fitted with transmitter units, for example an IR transmitter.

44. Facility as claimed in claim 43, **characterised in that** a receiver station for IR beams is disposed within range of the conveyor system (13).

45. Facility as claimed in one of claims 27 to 44, **characterised in that** at least one scanner (32), for example a reading device or similar, is provided on the conveyor system (13) for detecting the items.

46. Facility as claimed in one of claims 27 to 45, **characterised in that** the conveyor system (13) has at least one item stop (33).

47. Facility as claimed in claim 46, **characterised in that** an item stop (33) is mounted upstream of every change of direction in the conveyor system (13).

48. Facility as claimed in claim 46 or 47, **characterised in that** the operating unit for an item stop (33) preferably consists of a valve coil, two reed switches and a driver roller.

49. Facility as claimed in one of claims 27 to 48, **characterised in that** the conveyor system (13) has a counting station to identify items (5) that have been irradiated on one side.

50. Facility as claimed in claim 49, **characterised in that** the counting station preferably has three sensors.

51. Facility as claimed in one of claims 27 to 50, **characterised in that** the process conveyor (38) is preferably a wire mesh belt.

52. Facility as claimed in one of claims 27 to 51, **characterised in that** the facility (1) has at least one heat exchanger.

53. Facility as claimed in one of claims 27 to 52, **characterised in that** at least one venting system (17), preferably a fan, is provided in the irradiation chamber (16).

## Revendications

1. Procédé de modification des propriétés, au moins dans une zone partielle et/ou de traitement de produits (5), en particulier d'un matériau, d'un déchet, d'un composant, d'un produit alimentaire, d'un liquide, d'un gaz ou analogue, dans lequel les produits (5) sont transportés pour être passés, à l'aide d'un transporteur de processus (38) d'un système de manutention (13), à au moins un dispositif d'irradiation par des électrons, en particulier un accélérateur d'électrons, dans une chambre d'irradiation, les électrons nécessaires pour l'irradiation, sortant d'une cathode à incandescence, étant focalisés et étant pulsés, dans une unité accélératrice, avec des ondes d'une fréquence déterminée, susceptible d'être prédéfinie, en sortant, à une fréquence déterminée, hors du dispositif d'irradiation, et en étant dirigées sur les produits à irradier, **caractérisé en ce que** les produits sont transférés au transporteur de processus (38), depuis un transporteur d'accumulation (37), la vitesse du transfert étant prédéterminée, à l'aide d'un dispositif d'entraînement réglable, par une commande, de manière que les produits (5) se trouvant déjà sur le transporteur de processus (38) ne soient pas déplacés, et que les produits (5) soient, lors du transport, passés sur le dispositif d'irradiation par des électrons, sans qu'il y ait entre eux d'espaces intermédiaires.

2. Procédé selon la revendication 1, **caractérisé en ce qu'**une onde stationnaire est excitée dans l'unité accélératrice par une micro-onde pulsée.

3. Procédé selon la revendication 1 ou 2, **caractérisé en ce que** la vitesse d'avancement du transporteur de processus (38), la hauteur de balayage et le nombre des impulsions, ainsi que la durée d'impulsion, sont mutuellement adaptés, de manière que les impulsions de l'irradiation par des électrons se chevauchent d'une valeur d'au moins 30 %, de préférence 50 %.

4. Procédé selon l'une des revendications précédentes, **caractérisé en ce que** la vitesse d'avancement du transporteur de processus (38) est comprise entre 1 mm/s et 400 mm/s, de préférence entre 5 mm/s et 200 mm/s.

5. Procédé selon l'une des revendications précédentes, **caractérisé en ce que** l'on utilise plusieurs accélérateurs d'électrons pour irradier le produit (5).

6. Procédé selon l'une des revendications précédentes, **caractérisé en ce que** le produit (5) est irradié de façon alternée ou simultanée depuis plusieurs côtés, de préférence en direction verticale et/ou horizontale, si on se réfère au système de transport (13).

7. Procédé selon l'une des revendications précédentes, **caractérisé en ce qu'**un dosimètre, de préférence un dosimètre à film radiochrome, est utilisé pour mesurer la dose introduite dans le produit (5).

8. Procédé selon la revendication 7, **caractérisé en ce qu'**un dosimètre à transfert à alanine est utilisé pour l'établissement d'une courbe d'étalonnage, visant à évaluer les films.

9. Procédé selon la revendication 7 ou 8, **caractérisé en ce que**, après avoir procédé à l'évaluation des dosimètres à film par une mesure de l'intensité d'un rayon lumineux, traversant le dosimètre à film, dans la transmission, les valeurs caractéristiques appréhendées sont transférées à une installation informatique, dans le but d'un retraitement, d'un stockage et d'un contrôle.

10. Procédé selon l'une des revendications précédentes, **caractérisé en ce que** les doses de rayonnement agissant sur le produit (5) sont appréhendées par l'intermédiaire de dosimètres supplémentaires, de préférence des dosimètres calorimétriques.

11. Procédé selon l'une des revendications précédentes, **caractérisé en ce que** le produit (5) est passé, lors de son transport à l'aide d'un dispositif de transport (13), à une certaine vitesse, devant le point de sortie des électrons venant de l'unité accélératrice, en particulier la corne de balayage, qui s'oriente selon la dose à administrer.

12. Procédé selon l'une des revendications précédentes, **caractérisé en ce qu'**on place sur le produit (5) et/ou sur l'emballage du produit au moins un marquage, par exemple, un code à barres, identifié par un système d'identification, par exemple un scanneur (32), en particulier un lecteur.

13. Procédé selon l'une des revendications 7 à 12, **caractérisé en ce que**, sur le dosimètre utilisé de préférence, en particulier le dosimètre à film, on applique au moins un marquage visant à une évaluation, une identification et une vérification du dosimètre ultérieures, en particulier un marquage se présentant sous forme de code à barres, identifié par un système d'identification, par exemple, un scanneur, en particulier un lecteur.

14. Procédé selon la revendication 13, **caractérisé en ce que** le produit (5) est stocké en tampon sur un premier dispositif d'arrêt de produit (33), en aval du système d'identification.

15. Procédé selon l'une des revendications précédentes, **caractérisé en ce que** le produit (5), respectivement les produits (5), est (sont) stocké(s) en tampon en au moins deux endroits à peu près déterminés du système de transport (13), en particulier aux endroits auxquels se produit une modification de la direction de déplacement.

16. Procédé selon l'une des revendications précédentes, **caractérisé en ce que** le produit (5) est amené à un dispositif transporteur d'alimentation allant à l'enceinte d'irradiation (16), suivant des intervalles ayant été calculés à partir du degré de traitement.

17. Procédé selon l'une des revendications précédentes, **caractérisé en ce que** le degré de traitement du produit (5) est appréhendé par un scanneur (32), par exemple, un lecteur de code à barres ou analogue.

18. Procédé selon l'une des revendications précédentes, **caractérisé en ce que** le produit (5) est passé à plusieurs reprises sur le rayon d'électrons, par exemple en procédant à une modification répétée de la direction de déplacement.

19. Procédé selon l'une des revendications précédentes, **caractérisé en ce que** le produit (5) est prélevé du processus, par l'intermédiaire d'un dispositif de transport de prélèvement.

20. Procédé selon l'une des revendications précédentes, **caractérisé en ce que** le rayon d'électrons balaye la surface du produit (5) en suivant une courbe géométrique, qui est fixée par la combinaison de la vitesse d'avancement et du déplacement du rayon d'électrons, en particulier une courbe ayant une forme en dents de scie.

21. Procédé selon l'une des revendications précédentes, **caractérisé en ce que**, en au moins un produit (5), lors d'un essai préliminaire, la dose du rayon nécessaire est déterminée au moyen de dosimètres, montés en des endroits importants du produit (5), en particulier en des endroits auxquels la sollicitation par des rayons est faible et/ou élevée par suite du mode d'exploration adopté, par exemple au centre de la surface et/ou à l'intérieur du produit (5), aux angles du produit (5).

22. Procédé selon la revendication 21, **caractérisé en ce que** la valeur de la dose du rayon nécessaire, déterminée, est fournie à une unité de commande, par exemple une installation informatique qui, à l'aide de ces valeurs, détermine et surveille les vitesses nécessaires des différents dispositifs de transport.

23. Procédé selon l'une des revendications précédentes, **caractérisé en ce que**, pour effectuer le contrôle, pendant le processus de production, à des intervalles de temps définis, sur différents produits (5), on peut appliquer, de préférence sur la surface extérieure, des dosimètres, à l'aide desquels on peut contrôler le processus d'irradiation.

24. Procédé selon la revendication 23, **caractérisé en ce qu'**un système d'identification, associé à l'unité de commande, identifie les produits (5), munis des dosimètres.

25. Procédé selon l'une des revendications précédentes, **caractérisé en ce que** les données, obtenues à partir du processus d'irradiation de produits (5) spécifiques, sont amenées à un traitement informatique.

26. Procédé selon l'une des revendications précédentes, **caractérisé en ce que**, sur la base d'une comparaison THÉORIQUE/RÉELLE des doses introduites, on procède à la régulation de la vitesse des différentes parties de l'installation, en particulier du transporteur de processus (38) et/ou des réglages des paramètres du système accélérateur d'électrons.

27. Installation (1) pour modifier les propriétés, au moins dans une zone partielle, et/ou pour le traitement de produits (5) par un rayonnement, en particulier, d'un matériau, d'un déchet, d'un composant, d'un produit alimentaire, d'un liquide, d'un gaz ou analogue, avec un transporteur de processus (38), d'un système de transport (13), pour les produits (5) à traiter, avec une source de production d'électrons libres, avec un système accélérateur des électrons libres, avec une enceinte d'irradiation (16), dans laquelle les produits (5) sont irradiés et qui est entourée par des parois de protection contre les rayons, **caractérisée en ce que** le système de transport (13) est réalisé de manière que les produits (5) à irradier, dans le but d'augmenter l'efficacité de l'installation (1), peuvent être passés, lors du transport, sans espace intermédiaire entre eux, sur le dispositif de sortie d'électrons (14), depuis l'unité accélératrice, sachant qu'un transporteur d'accumulation (37) est disposé en amont du transporteur de processus (38), dans la direction de transport, et est muni d'un dispositif d'entraînement réglé par une commande.

28. Installation selon la revendication 27, **caractérisée en ce que** l'amenée et l'évacuation des produits (5) dans et hors de l'enceinte d'irradiation (16) se fait en différents côtés des parois de protection contre les rayons, en passant chaque fois par des accès propres, du genre de labyrinthes, permettant l'amenée et l'évacuation des produits (5).

29. Installation selon la revendication 27 ou 28, **caractérisée en ce que**, au moins en un endroit du système de transport (13), est prévu un dispositif, par exemple, une butée en métal et/ou en matière synthétique ou analogue, de sorte que s'effectue une orientation automatique des produits (5), que ceux-ci puissent être passés, lors du transport, sur le rayon d'électrons, en étant placés sous un angle défini par rapport au dispositif de sortie d'électrons (14), cet angle étant, de préférence, de 90°.

30. Installation selon l'une des revendications 27 à 29, **caractérisée en ce que** le système de transport (13), à l'extérieur de la zone à risque de l'accélérateur d'électrons, est formé de deux zones séparées, sachant que la zone d'alimentation non pure (24) est séparée d'une zone d'évacuation pure (26) au moyen d'un dispositif d'isolement (25), par exemple une grille d'isolement, une paroi d'isolement.

31. Installation selon l'une des revendications 27 à 30, **caractérisée en ce que** l'accélérateur est un accélérateur annulaire.

32. Installation selon l'une des revendications 27 à 31, **caractérisée en ce que** le dispositif de sortie d'électrons (14) est un cornet de balayage, ayant une hauteur de balayage allant jusqu'à 100 cm, de préférence de 60 cm.

33. Installation selon l'une des revendications 27 à 31, **caractérisée en ce que** le dispositif de sortie d'électrons (14) est guidé autour du système de transport (13), en adoptant la forme d'un cercle.

34. Installation selon l'une des revendications 27 à 33, **caractérisée en ce que** l'accélérateur d'électrons est réalisé de manière que la dose, déposée dans le produit (5), soit largement indépendante de la profondeur de pénétration dans le produit (5), jusqu'à une profondeur de pénétration du rayon d'électrons de 50 cm, de préférence de 30 cm.

35. Installation selon l'une des revendications 27 à 34, **caractérisée en ce que**, sur le système de transport (13), pour obtenir une distribution de dose homogène dans le produit (5), on peut régler une vitesse d'avancement, permettant un chevauchement des impulsions d'au moins 30 %, de préférence de 50 %.

36. Installation selon l'une des revendications 27 à 35, **caractérisée en ce que** l'unité accélératrice est disposée de manière à ce que l'irradiation se fasse par le dessus, respectivement par le dessous.

37. Installation selon l'une des revendications 27 à 36, **caractérisée en ce que** le système de transport (13) est réalisé de manière à permettre une irradiation simultanée du produit (5) depuis plusieurs côtés.

38. Installation selon l'une des revendications 27 à 37, **caractérisée en ce que**, sur le système de transport (13), peut être réglée une vitesse d'avancement de 1 mm/sec jusqu'à 400 mm/sec, de préférence 5 mm/sec à 200 mm/sec.

39. Installation selon l'une des revendications 27 à 38, **caractérisée en ce que** le système de transport (13) est formé de transporteurs continus, par exemple une bande à rouleaux et/ou un transporteur à chaîne.

40. Installation selon l'une des revendications 27 à 39, **caractérisée en ce que** le système de transport (13) est formée d'une bande d'alimentation (28), d'une bande d'amenée (34), d'un transporteur de processus (38), d'un transporteur accumulateur (37), d'un transporteur élévateur (40), d'un transporteur transversal (36, 42), d'au moins un retoumeur d'angle et d'une bande de réception (43).

41. Installation selon l'une des revendications 27 à 40, **caractérisée en ce que**, sur le système de transport (13), est disposé au moins un dispositif de marquage (31) pour le produit (5), par exemple un distributeur d'étiquettes, un dispositif d'application de micro-puces électroniques, ou analogues.

42. Installation selon la revendication 41, **caractérisée en ce que** les étiquettes sont munies d'un code à barres.

43. Installation selon la revendication 41, **caractérisée en ce que** les micro-puces utilisées sont équipées d'installations émettrices, par exemple d'un émetteur IR.

44. Installation selon la revendication 43, **caractérisée en ce qu'**une station de réception des rayons IR est disposée dans la zone du système de transport (13).

45. Installation selon l'une des revendications 27 à 44, **caractérisée en ce que**, sur le système de transport (13), est monté au moins un scanneur (32), par exemple un lecteur ou analogue, afin d'assurer la détection des produits.

46. Installation selon l'une des revendications 27 à 45, **caractérisée en ce que** le système de transport (13) comprend au moins un dispositif d'arrêt de produit (33).

47. Installation selon la revendication 46, **caractérisée en ce qu'**un dispositif d'arrêt de produit (33) est monté avant chaque changement de direction du système de transport (13).

48. Installation selon la revendication 46 ou 47, **caractérisée en ce que** le dispositif d'actionnement d'un dispositif d'arrêt (33) est formé, de préférence, d'une bobine de soupape, de deux interrupteurs à tiges (reed) et d'un galet d'entraînement.

49. Installation selon l'une des revendications 27 à 48, **caractérisée en ce que** le système de transport (13) comprend un poste de comptage, visant à déterminer les produits (5) ayant été irradiés sur un côté.

50. Installation selon la revendication 49, **caractérisée en ce que** le poste de comptage comprend de préférence trois capteurs.

51. Installation selon l'une des revendications 27 à 50, **caractérisée en ce que** le transporteur de processus (38) est réalisé de préférence sous la forme de courroies tressées en fil métallique.

52. Installation selon l'une des revendications 27 à 51, **caractérisée en ce que** l'installation (1) comprend au moins un échangeur de chaleur.

53. Installation selon l'une des revendications 27 à 52, **caractérisée en ce qu'**au moins un dispositif de désaération (17), par exemple un ventilateur, est disposé dans l'enceinte d'irradiation (16).
